# EUROPEAN PATENT APPLICATION

(11) **EP 4 796 127 A1**
(43) Date of publication of application: **26.08.2026**
(21) Application number: 24884961.4
(22) Date of filing: 01.11.2024
(51) Int. Cl.: A61K 47/64, A61K 47/66, A61P 35/00, C12N 15/11

(54) **OLIGONUCLEOTIDE-BASED PROTAC MOLECULE FOR TARGETED DEGRADATION OF LIN28A/B AND USE THEREOF**

(30) Priority: 03.11.2023 CN 202311457778
(71) Applicant: Peking University, Beijing 100871 (CN)
(72) Inventor: TANG, Xinjing, Beijing 100871 (CN); XU, Jianfei, Beijing 100871 (CN)
(74) Representative: Bayramoglu et al.
(86) International application number: PCT/CN2024/129316
(87) International publication number: WO 2025/092964

(57) **Abstract**

The present disclosure discloses an oligonucleotide-based proteolysis-targeting chimera (PROTAC) molecule for targeted degradation of a Lin28A/B protein, and a use thereof. The oligonucleotide-based PROTAC molecule of the present disclosure is prepared by coupling an oligonucleotide sequence capable of binding to the Lin28A/B protein to a ligand capable of promoting ubiquitination of the Lin28A/B protein through any of linkers of different types or different lengths. In different tumor cells, the oligonucleotide-based PROTAC molecule can achieve targeted degradation of the Lin28A/B protein, exhibit significant degradation activity against the Lin28A/B protein, and exert an anti-tumor function. The present disclosure demonstrates promising application prospects in the preparation of drugs or formulations for targeted degradation of the Lin28A/B protein, in the preparation of anti-tumor drugs or formulations, and in the preparation of combination therapies with small-molecule or large-molecule anti-tumor drugs.

## Description

### TECHNICAL FIELD

The present disclosure relates to a proteolysis-targeting chimera (PROTAC) and a use thereof, and in particular, relates to an oligonucleotide-based PROTAC molecule for targeted degradation of a Lin28A/B protein, and a use of the oligonucleotide-based PROTAC molecule in preparation of a drug or a formulation for targeted degradation of a Lin28A/B protein or for an anti-tumor therapy or in preparation of a drug used in combination with an anti-tumor drug. The present disclosure belongs to the field of PROTACs and applications thereof.

### BACKGROUND TECHNOLOGY

Structurally, Lin28 is an evolutionarily highly conserved RNA-binding protein, including two RNA-binding domains (a cold shock domain (CSD) and a CCHC zinc finger domain). Lin28 was initially identified as a key regulator of developmental timing in *Caenorhabditis elegans.* Lin28 (Lin28A and Lin28B) can maintain cellular stemness in mammals. Lin28 is highly expressed in embryonic cells and promotes rapid and substantial cell proliferation. Lin28 plays an important role in various processes such as embryogenesis, skeletal myogenesis, germ cell development, and gliogenesis. Accumulating evidence suggests that Lin28 may also serve as a key regulator controlling the pluripotency of embryonic stem (ES) cells. Lin28, together with OCT4, SOX2, and NANOG (reprogramming factors), can reprogram somatic cells into induced pluripotent stem cells. Lin28 can bind to mRNAs, regulating the stability and translation of mRNAs. Additionally, Lin28 can bind to the terminal loop of the miRNA let-7 precursor, thereby preventing the processing of let-7 into a mature form. It has been demonstrated in human tumors that Lin28 is overexpressed in various malignant tumors and can significantly promote the proliferation, migration, and drug resistance of tumor cells. Lin28 functions as an oncogene in malignant transformation and tumor progression and is closely associated with poor prognosis in cancer therapy. Lin28 has two isoforms, Lin28A and Lin28B, which are localized in the cytoplasm and nucleus of tumor cells, respectively. The relative levels of Lin28A and Lin28B vary among different types of tumor cells. However, both isoforms of Lin28 can promote the proliferation, migration, and drug resistance of tumor cells. Therefore, inhibiting Lin28 expression represents a novel cancer treatment strategy.

PROTAC technology originated from the discovery of ubiquitin (Ub)-regulated protein degradation by scientists. Eukaryotic cells constantly strive to maintain appropriate protein levels, as thousands of proteins are being synthesized and degraded at every moment. A key factor in maintaining protein homeostasis is a small protein molecule known as Ub. Once linked to proteins, Ub leads to the transport of these proteins to proteasomes for degradation. PROTAC is a heterobifunctional molecule composed of the following three parts: (1) a ligand that binds to a target protein; (2) a ligand that recruits an E3 Ub ligase to facilitate ubiquitination of the target protein; and (3) a linker that connects these ligands. As a new and promising technology, PROTACs demonstrate great potential in the following aspects: PROTACs exhibit special sensitivity to drug-resistant targets. Traditionally, chemotherapy is the major approach for cancer treatment. Acquired resistance to chemotherapeutic drugs hinders clinical applications of the chemotherapeutic drugs and leads to disease relapse. With advances in research on new targets and new drug discovery technologies, another powerful strategy is to directly and specifically inhibit the function of an oncogenic protein or receptor with a small molecule. It should be noted that the discovery of kinase inhibitors has not resolved the issue of drug resistance in cancer therapy. In contrast, PROTACs affect protein functions by eliminating the entire target to remove all functions of the target, including both enzymatic and non-enzymatic activities. As a result, PROTACs can address the potential drug resistance faced by current therapies. Further, due to catalysis, PROTACs are less sensitive to increased target expression and target protein mutations. Only low doses of PROTACs are required to achieve excellent efficacy. PROTACs enable deep and sustained degradation of target proteins, provide rapid and potent inhibition of downstream signals, and exert long-lasting suppression on tumor initiation and progression *in vitro.* Consequently, the use of PROTACs for protein degradation has become a hot spot in current cancer therapy.

### CONTENT OF THE INVENTION

A first objective of the present disclosure is to provide an oligonucleotide-based PROTAC molecule for targeted degradation of a Lin28A/B protein.

A second objective of the present disclosure is to provide a use of the oligonucleotide-based PROTAC molecule in preparation of a drug or formulation for targeted degradation of a Lin28A/B protein or for an anti-tumor therapy or in a combination therapy.

To achieve the above objectives, the present disclosure adopts the following technical solutions:

In an aspect of the present disclosure, an oligonucleotide-based PROTAC molecule for targeted degradation of a Lin28A/B protein is provided, where the oligonucleotide-based PROTAC molecule is produced by coupling, through a linker, an oligonucleotide sequence capable of binding to the Lin28A/B protein to a ligand capable of recruiting an E3 Ub ligase to promote ubiquitination of a target protein. A method for the coupling may be as follows: preparing an oligonucleotide sequence that is capable of binding to the Lin28A/B protein and is modified with an amino group at a 3' terminus through solid-phase synthesis, and crosslinking the oligonucleotide sequence with an N-hydroxysuccinimide (NHS) active ester of a CRBN or von Hippel-Lindau (VHL) ligand linked to any of different linkers through the amino group to produce the oligonucleotide-based PROTAC molecule.

In a preferred specific embodiment of the present disclosure, the oligonucleotide sequence capable of binding to the Lin28A/B protein can be an oligonucleotide sequence that binds to both CSD and a zinc knuckle domain (ZKD) of the Lin28A/B protein, or an oligonucleotide sequence that binds only to the CSD of the Lin28A/B protein, or an oligonucleotide sequence that binds only to the ZKD of the Lin28A/B protein; preferably, the oligonucleotide sequence that binds to both the CSD and the ZKD of the Lin28A/B protein is any nucleotide sequence selected from SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, or SEQ ID No. 23; the oligonucleotide sequence that binds only to the CSD of the Lin28A/B protein is any nucleotide sequence selected from SEQ ID No. 2, SEQ ID No. 4, SEQ ID No. 6, SEQ ID No. 8, SEQ ID No. 10, SEQ ID No. 12, SEQ ID No. 14, SEQ ID No. 16, SEQ ID No. 18, SEQ ID No. 20, SEQ ID No. 22, or SEQ ID No. 24; or the oligonucleotide sequence that binds to both the CSD and the ZKD of the Lin28A/B protein or the oligonucleotide sequence that binds only to the CSD of the Lin28A/B protein is a nucleotide sequence that has a homology of 80% or more with the above respective nucleotide sequence and still retains a function or an activity of binding to the Lin28A/B protein.

The oligonucleotide sequence that binds only to the ZKD of the Lin28A/B protein is any nucleotide sequence selected from (1) to (12):
(1): 5'-GGGAGAU-3'; (2): 5'-GGGAGAU-3'; (3): 5'-AUGGGAU-3'; (4): 5'-GGAAGAU-3'; (5): 5'-GGGAGUU-3'; (6): 5'-AGGAGGU-3' ; (7): 5'-AGGAGAU-3' ; (8): 5'-AGGAGAU-3'; (9): 5'-UGGAGAU-3'; (10): 5'-AGGAGAU-3'; (11): 5'-UGGAGAU-3'; (12): 5'-AGAAGAU-3'.

Further preferably, any of the nucleotide sequences defined above may also be subjected to a full-backbone thio modification. The nucleotide sequences may have different nucleotide analog modifications, including, but not limited to, the following modified nucleobases: 5-methylcytosine, 6-methyladenine, ribavirin, pseudouracil, and/or inosine; or a nucleotide having a methoxy, methylethoxy, or fluoro modification at a 2' position of a sugar ring, or a locked nucleic acid.

In the present disclosure, the ligand capable of recruiting an E3 Ub ligase to promote ubiquitination of a target protein is preferably a CRBN ligand (pomalidomide), a VHL ligand (VH032), or a derivative or an analog thereof. A chemical name of the CRBN ligand, a small molecule, is as follows: 4-amino-2-(2,6-dioxo-3-piperidinyl)-1H-isoindole-1,3(2H)-dione. A structural formula of the CRBN ligand is shown in a formula I:

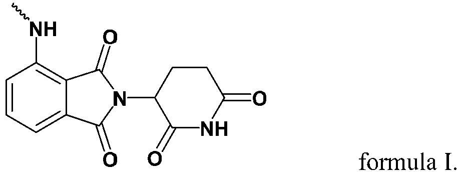

In the present disclosure, a chemical name of the VHL ligand, a small molecule, is as follows:
(2S,4R)-1-((S)-2-amino-3,3-dimethylbutyl)-4-hydroxy-N-(4-(4-methylthiazol-5-yl)benzyl)pyrrolidi ne-2-carboxamide. A structural formula of the VHL ligand is shown in a formula II:

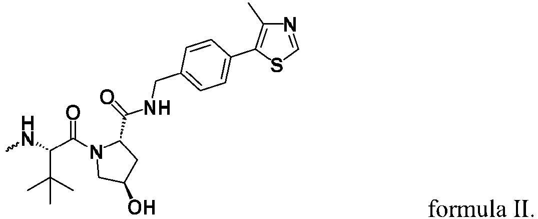

A structural formula of the derivative or the analog in the present disclosure is shown in a formula III:

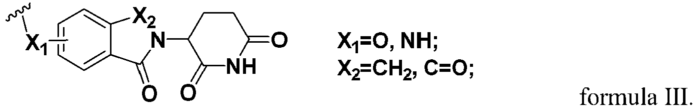

In the present disclosure, the linker includes, but is not limited to, one or more of alkylene, alkoxy, alkylamino, alkylthio, amido, a carbamate linkage, or a triazole derivative.

Preferably, the linker is selected from any one of compounds shown in the following general formulas: 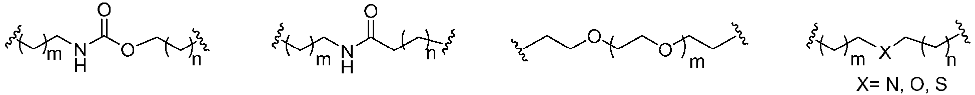 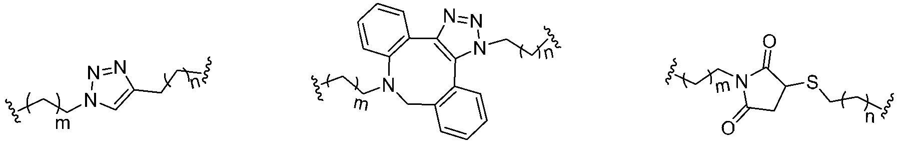 where m or n is any integer from 0 to 10.

In a more preferred specific embodiment, the linker includes, but is not limited to, any one of C3, C5, C6, C8, C12-1, C12-2, C16, P4, or P6:

| Linker | Structural formula |
|---|---|
| C3 | 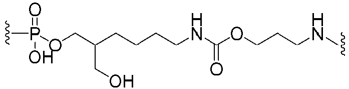 |
| C6 | 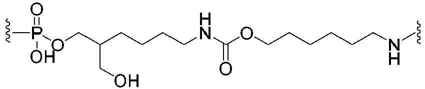 |
| C12-1 | 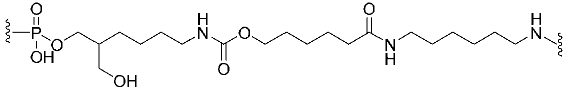 |
| C16 | 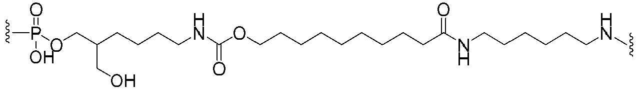 |
| P4 | 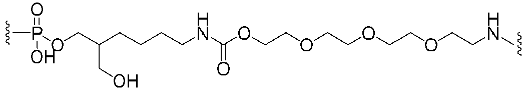 |
| P6 | 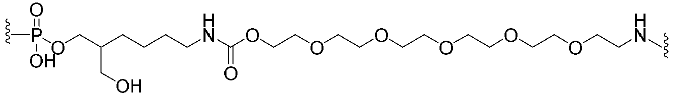 |
| C5 | 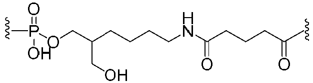 |
| C8 | 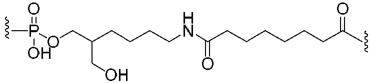 |
| C12-2 | 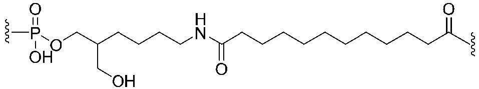 |

Another aspect of the present disclosure provides a method for preparing the oligonucleotide-based PROTAC molecule for targeted degradation of a Lin28A/B protein, including:
(1) linking a CRBN or VHL ligand to any of linkers of different types or different lengths, and conducting an activation reaction to produce an NHS active ester of the CRBN or VHL ligand with a linker; and
(2) coupling the oligonucleotide sequence that is capable of binding to the Lin28A/B protein and includes an amino group at a 3' terminus to the NHS active ester of the CRBN or VHL ligand with the linker obtained in the step (1).

As a preferred embodiment, the step (2) includes: dissolving the oligonucleotide sequence that is capable of binding to the Lin28A/B protein and includes the amino group at the 3' terminus in phosphate buffered saline (PBS) to produce a mixed solution 1; dissolving the NHS active ester of the CRBN or VHL ligand with the linker in dimethyl sulfoxide (DMSO) to produce a mixed solution 2; and mixing the mixed solution 1 and the mixed solution 2 to allow a coupling reaction, and purifying a reaction product.

As a preferred embodiment, in the step (2), a concentration ratio of the oligonucleotide sequence that is capable of binding to the Lin28A/B protein and includes the amino group at the 3' terminus to the NHS active ester of the CRBN or VHL ligand with the linker is 1:500.

The oligonucleotide-based PROTAC molecule for targeted degradation of a Lin28A/B protein provided by the present disclosure exhibits significant degradation activity against both Lin28A and Lin28B proteins. The degradation effect of an oligonucleotide-based PROTAC molecule group is significantly superior to degradation effects of a Full-S ORN experimental group without coupled pomalidomide and a Full-ORN/pomalidomide mixture group. Moreover, an experimental group treated with pomalidomide alone does not show significant degradation of the Lin28A protein. It can be seen that the degradation of the Lin28A or Lin28B protein by the oligonucleotide-based PROTAC molecule of the present disclosure is specific degradation of Lin28A/B mediated jointly by the oligonucleotide sequence capable of binding to Lin28A/B and the derivative of the pomalidomide or VH that are coupled through a linker, rather than non-specific degradation mediated by any single one of the aforementioned components alone.

According to experimental results of Lin28A/B degradation or miRNA let-7 level changes mediated by the oligonucleotide-based PROTAC molecule of the present disclosure, as the Lin28 protein is degraded, the expression level of any mature let-7 is upregulated, while the expression level of miR-21 remains unchanged. It indicates that the targeted degradation of Lin28A/B specifically leads to an increase in the expression level of miRNA let-7, a tumor suppressor. Under the same transfection concentration and time, the oligonucleotide-based PROTAC molecule of the present disclosure exhibits a better effect on increasing let-7g levels than the Full-S ORN experimental group without coupled pomalidomide and the Full-ORN/pomalidomide mixture group, while the experimental group treated with pomalidomide alone does not show a significant increase in let-7g levels.

In the present disclosure, to enhance the targeting capability of the oligonucleotide-based PROTAC molecule against tumor cells, a targeting moiety may be introduced at a terminus of a nucleotide sequence of the oligonucleotide-based PROTAC molecule. The targeting moiety includes, but is not limited to, folic acid, a polypeptide, or a nucleic acid aptamer.

The oligonucleotide-based PROTAC of the present disclosure can significantly inhibit the proliferation, colony formation, migration, and invasion of T47D or H1299 cells. The corresponding inhibitory effect can be further enhanced when the oligonucleotide-based PROTAC is used in combination with a small-molecule drug.

Thus, the oligonucleotide-based PROTAC provided by the present disclosure can be used in the following aspects:
(1) Preparation of a drug or formulation for targeted degradation of the Lin28A/B protein.
(2) Preparation of a drug or formulation for an anti-tumor therapy: Lin28A/B is overexpressed in tumor cells targeted by the anti-tumor therapy.
(3) Use in combination with a small-molecule anti-tumor drug for an anti-tumor therapy: The small-molecule anti-tumor drug may be tamoxifen or paclitaxel.

In the present disclosure, an oligonucleotide sequence capable of binding to the Lin28A/B protein is coupled to a ligand capable of promoting ubiquitination of the Lin28A/B protein through any of linkers of different types or different lengths to produce an oligonucleotide-based PROTAC molecule. Such an oligonucleotide-based PROTAC molecule can be used in different tumor cells to achieve selective degradation of Lin28A and/or Lin28B proteins and play an anti-tumor role. The present disclosure demonstrates promising application prospects in the preparation of drugs or formulations for targeted degradation of the Lin28A/B protein, in the preparation of anti-tumor drugs, or in combination therapies with anti-tumor drugs such as small-molecule anti-tumor drugs or macromolecular anti-tumor antibodies for tumors.

### DESCRIPTION OF THE DRAWINGS

FIG. 1 shows the degradation of Lin28A/B by PROTAC molecules prepared from different miRNA sequences targeting Lin28, where a): Lin28A (T47D cells), b): Lin28B (H1299 cells), and c): Lin28B (Huh7 cells);
FIG. 2 shows the degradation of a Lin28A protein by Let-7f-1-based PROTAC molecules in T47D cells, where a): full-length-sequence-targeting sequence, b): CSD-targeting sequence, c): ZKD-targeting sequence, d): concentration dependence, e): time dependence, and f): specificity;
FIG. 3 shows Let-7 expression level changes in T47D (a to d)/H1299 (e to f) cells after degradation of a Lin28A/B protein by Let-7f-1 Full-S P4-C, where a): evaluation of upregulation effects for all Let-7 in T47D cells, b): concentration dependence, c): time dependence, d): specificity, e): evaluation of upregulation effects for all Let-7 in H1299 cells, f): concentration dependence, g): time dependence, and h): specificity;
FIG. 4 shows an anti-tumor activity of Let-7f-1 Full-S P4-C in T47D cells, where a): cell proliferation, b): combination therapy, c): cell cycle, d): colony formation, e): apoptosis, and f): cell migration;
FIG. 5 shows the degradation of the Lin28B protein by Let-7f-1-based PROTAC molecules in H1299 cells, where a): full-length-sequence-targeting sequence, b): CSD-targeting sequence, c): ZKD-targeting sequence, d): concentration dependence, e): time dependence, and f): specificity;
FIG. 6 shows an anti-tumor activity of Let-7f-1 Full-S P4-C in H1299 cells, where a): cell proliferation, b): colony formation, c): cell migration, d): cell invasion, and e): apoptosis;
FIG. 7 shows the degradation of the Lin28B protein by Let-7b-based PROTAC molecules in Huh7 cells, where a): full-length-sequence-targeting sequence, b): CSD-targeting sequence, c): ZKD-targeting sequence, d): concentration dependence, e): time dependence, and f): specificity; and
FIG. 8 shows the *in vivo* anti-tumor activity evaluation of a PROTAC molecule Let-7f-1 Full-S P4-C, where a): body weight changes in mice, b): tumor volume growth curves, c): normal tissue weights in mice, d): photographs of tumors dissected from mice, e): tumor weights, f): Lin28A expression levels in tumors, and g): tumor tissue sections.

### SPECIFIC IMPLEMENTATIONS

The present disclosure will be further described below in conjunction with specific embodiments, and the advantages and features of the present disclosure will become clear from the description. However, these embodiments are only exemplary and do not constitute any limitation to the scope of the present disclosure. Those skilled in the art should appreciate that modifications and substitutions can be made to the details and forms of the present disclosure without departing from the spirit and scope of the present disclosure, but these modifications and substitutions fall within the scope of protection of the present disclosure.

### Example 1 Design of oligonucleotide sequences capable of binding to Lin28A/B

The oligonucleotide sequences capable of binding to Lin28A/B are as follows:
Let-7a-1 Full-S: 5'-UUAGGGUCACACCCACCACUGGGAGAU-3' (SEQ ID No. 1)
Let-7a-1 CSD-S: 5'-UUAGGGUCACACCCACCACUG-3' (SEQ ID No. 2)
Let-7a-2 Full-S: 5'-UAGAAUUACAUCAAGGGAGAU-3' (SEQ ID No. 3)
Let-7a-2 CSD-S: 5'-UAGAAUUACAUCAAG-3' (SEQ ID No. 4)
Let-7a-3 Full-S: 5'-UGGGGCUCUGCCCUGCUAUGGGAU-3' (SEQ ID No. 5)
Let-7a-3 CSD-S: 5'-UGGGGCUCUGCCCUGCUA-3' (SEQ ID No. 6)
Let-7b Full-S: 5'-UCAGGGCAGUGAUGUUGCCCCUCGGAAGAU-3' (SEQ ID No. 7)
Let-7b CSD-S: 5'-UCAGGGCAGUGAUGUUGCCCCUCG-3' (SEQ ID No. 8)
Let-7c Full-S: 5'-UAGAGUUACACCCUGGGAGUU-3' (SEQ ID No. 9)
Let-7c CSD-S: 5'-UAGAGUUACACCCUG-3' (SEQ ID No. 10)
Let-7d Full-S: 5'-UUAGGGCAGGGAUUUUGCCCACAAGGAGGU-3' (SEQ ID No. 11)
Let-7d CSD-S: 5'-UUAGGGCAGGGAUUUUGCCCACAA-3' (SEQ ID No. 12)
Let-7e Full-S: 5'-GAGGAGGACACCCAAGGAGAU-3' (SEQ ID No. 13)
Let-7e CSD-S: 5'-GAGGAGGACACCCAA-3' (SEQ ID No. 14)
Let-7f-1 Full-S: 5'-GUGGGGUAGUGAUUUUACCCUGUUCAGGAGAU-3'(SEQ ID No. 15)
Let-7f-1 CSD-S: 5'-GUGGGGUAGUGAUUUUACCCUGUUCA-3' (SEQ ID No. 16)
Let-7f-2 Full-S: 5'-UUAGGGUCAUACCCCAUCUUGGAGAU-3' (SEQ ID No. 17)
Let-7f-2 CSD-S: 5'-UUAGGGUCAUACCCCAUCUU-3' (SEQ ID No. 18)
Let-7g Full-S: 5'-UGAGGGUCUAUGAUACCACCCGGUACAGGAGAU-3' (SEQ ID No. 19)
Let-7g CSD-S: 5'-UGAGGGUCUAUGAUACCACCCGGUACA-3' (SEQ ID No. 20)
Let-7i Full-S: 5'-GGUCGGGUUGUGACAUUGCCCGCUGUGGAGAU-3' (SEQ ID No. 21)
Let-7i CSD-S: 5'-GGUCGGGUUGUGACAUUGCCCGCUGU-3' (SEQ ID No. 22)
miR98 Full-S: 5'-GUGGGGUAGGGAUAUUAGGCCCCAAUUAGAAGAU-3' (SEQ ID No. 23)
miR98 CSD-S: 5'-GUGGGGUAGGGAUAUUAGGCCCCAAUU-3' (SEQ ID No. 24)
Let-7a-1 ZKD-S: 5'-GGGAGAU-3'
Let-7a-2 ZKD-S: 5'-GGGAGAU-3'
Let-7a-3 ZKD-S: 5'-AUGGGAU-3'
Let-7b ZKD-S: 5'-GGAAGAU-3'
Let-7c ZKD-S: 5'-GGGAGUU-3'
Let-7d ZKD-S: 5'-AGGAGGU-3'
Let-7e ZKD-S: 5'-AGGAGAU-3'
Let-7f-1 ZKD-S: 5'-AGGAGAU-3'
Let-7f-2 ZKD-S: 5'-UGGAGAU-3'
Let-7g ZKD-S: 5'-AGGAGAU-3'
Let-7i ZKD-S: 5'-UGGAGAU-3' and
miR98 ZKD-S: 5'-AGAAGAU-3'.

The above oligonucleotide sequences may be subjected to a full-backbone thio modification, that is, non-bridging oxygen atoms in the original phosphate bonds may be substituted with sulfur atoms.

### Example 2 Synthesis and characterization of CRBN ligand small molecules (pomalidomide) linked to linkers of different lengths

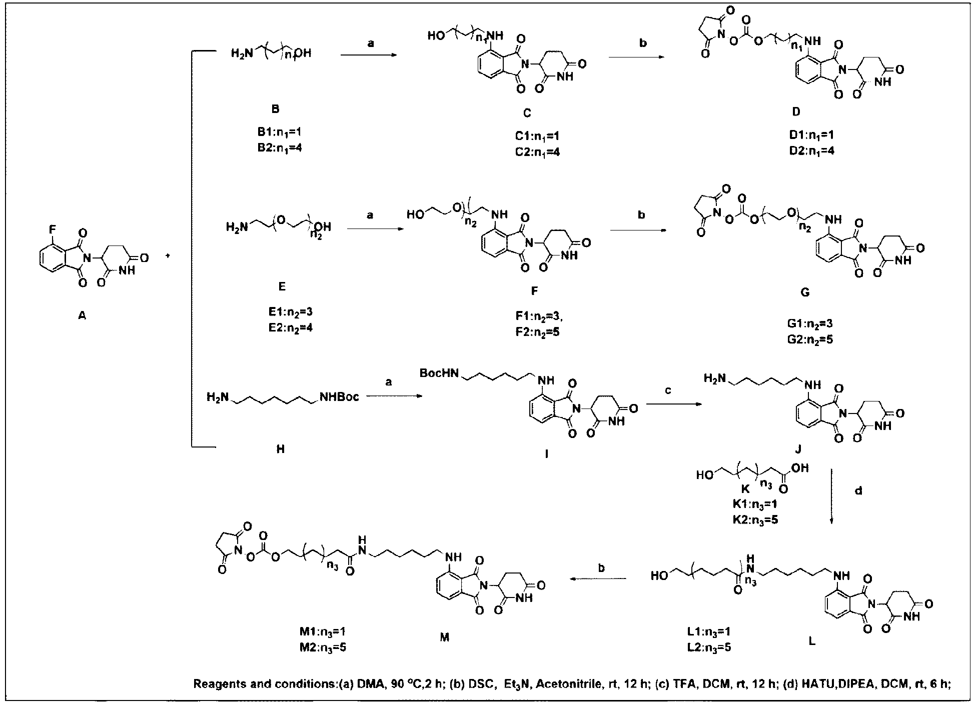

### Synthetic routes were as follows:

1) Synthesis of compounds C: 2-(2,6-dioxopiperidin-3-yl)-4-fluoroindoline-1,3-dione (1 mmol, 1.0 equiv) was dissolved in N,N-dimethylacetamide (2 mL), and 3-amino-1-propanol (1.2 mmol, 1.2 equiv) or 6-amino-1-hexanol (1.2 mmol, 1.2 equiv) was added. The resulting reaction mixture was heated to 90°C and stirred for 2 h, and then subjected to extraction with ethyl acetate and water. The resulting crude product was purified by silica gel column chromatography to produce the compounds C.
2) Synthesis of compounds D (NHS active esters of a small-molecule CRBN ligand that included linkers C3 and C6, respectively): A compound C (0.2 mmol, 1.0 equiv) and N,N'-disuccinimidyl carbonate (0.3 mmol, 1.5 equiv) were dissolved in anhydrous acetonitrile, and then triethylamine (0.6 mmol, 3.0 equiv) was added. The resulting mixture was stirred at room temperature for 16 h, subjected to solvent evaporation, subjected to extraction with dichloromethane and water, washed with a cold 5% sodium bicarbonate solution, dried over anhydrous sodium sulfate, and purified by silica gel column chromatography to produce the compounds D1 and D2.
   D1(C3-C): ¹H NMR (400 MHz, CDCl₃) δ 8.07 (s, 1H), 7.57 (t, J = 8.5, 7.2 Hz, 1H), 7.16 (d, J = 7.1 Hz, 1H), 6.95 (d, J = 8.5 Hz, 1H), 4.94 (dd, J = 12.1, 5.3 Hz, 1H), 4.49 (t, J = 5.9 Hz, 2H), 3.50 (t, J = 6.8 Hz, 3H), 2.96 - 2.89 (m, 1H), 2.88 (s, 4H), 2.85 - 2.78 (m, 2H), 2.21 - 2.16 (m, 2H), 2.13 (m, 3H). ¹³C NMR (101 MHz, CDCl₃) δ 171.27, 169.55, 168.54, 167.66, 146.98, 136.13, 132.50, 116.70, 111.43, 109.88, 62.72, 48.88, 42.59, 32.52, 31.42, 29.15, 26.72, 25.48, 22.81. ESI: [M + H]⁺ cacld: 473.1230, found: 473.1303. [M + Na]⁺ found: 495.1122.
   D2(C6-C): ¹H NMR (400 MHz, CDCl₃) δ 8.07 (s, 1H), 7.52 (t, *J* = 8.5, 7.2 Hz, 1H), 7.11 (d, *J= 7.0* Hz, 1H), 6.91 (d, *J=* 8.5 Hz, 1H), 4.93 (dd, *J=* 12.1, 5.3 Hz, 1H), 4.36 (t, *J=* 6.5 Hz, 3H), 3.31 (d, *J* = 4.6 Hz, 3H), 2.95 - 2.88 (m, 1H), 2.86 (s, 4H), 2.82 - 2.72 (m, 2H), 2.22 - 2.12 (m, 1H), 1.86 - 1.77 (m, 3H), 1.76 - 1.67 (m, 3H), 1.49 (m, 5.4 Hz, 5H). ¹³C NMR (101 MHz, CDCl₃) δ 170.93, 169.50, 168.72, 168.26, 167.63, 151.63, 146.98, 136.16, 132.52, 116.67, 111.48, 109.97, 71.34, 48.89, 42.45, 31.42, 29.00, 28.28, 26.42, 25.49, 25.22, 22.82. ESI: [M + H]⁺ cacld: 515.1700, found: 515.1769. [M + Na]⁺ found: 537.1597.
3) Synthesis of compounds F: 2-(2,6-dioxopiperidin-3-yl)-4-fluoroindoline-1,3-dione (1 mmol, 1.0 equiv) was dissolved in N,N-dimethylacetamide (2 mL), and 1-amino-3,6,9-trioxa-11-undecanol or 17-amino-3,6,9,12,15-pentaoxaheptadecanol (1.2 mmol, 1.2 equiv) was added. The resulting reaction mixture was heated to 90°C and stirred for 2 h, and then subjected to extraction with ethyl acetate and water. The resulting crude product was purified by silica gel column chromatography to produce the compounds F.
4) Synthesis of compounds G (NHS active esters of a small-molecule CRBN ligand that included linkers P4 and P6, respectively): A compound F (0.2 mmol, 1.0 equiv) and N,N'-disuccinimidyl carbonate (0.3 mmol, 1.5 equiv) were dissolved in anhydrous acetonitrile, and then triethylamine (0.6 mmol, 3.0 equiv) was added. The resulting mixture was stirred at room temperature for 16 h, subjected to solvent evaporation, subjected to extraction with dichloromethane and water, washed with a cold 5% sodium bicarbonate solution, dried over anhydrous sodium sulfate, and purified by silica gel column chromatography to produce the compounds G1 and G2.
   G1(P4-C): G1: 1H NMR (400 MHz, CDCl₃) δ 8.30 (s, 1H), 7.50 (t, *J =* 8.5, 7.2 Hz, 1H), 7.11 (d, *J* = 7.1 Hz, 1H), 6.94 (d, *J* = 8.5 Hz, 1H), 4.93 (dd, *J* = 12.0, 5.2 Hz, 1H), 4.47(m, 2H), 3.82 - 3.78 (m, 2H), 3.75 (t, *J=* 5.3 Hz, 2H), 3.70 (d, *J* = 4.8 Hz, 6H), 3.52 - 3.47 (m, 2H), 2.91 (s, 1H), 2.85 (s, 4H), 2.81 - 2.71 (m, 2H), 2.16 - 2.10 (m, 1H). ¹³C NMR (101 MHz, CDCl₃) δ 171.23, 169.26, 168.71, 168.42, 167.67, 151.68, 146.87, 136.04, 132.52, 129.61, 116.83, 115.31, 111.61, 110.27, 70.85, 70.75, 70.72, 70.34, 69.48, 48.87, 42.42, 31.41, 25.48, 22.74. ESI: [M + H]⁺ cacld: 591.1860, found: 591.1874. [M + Na]⁺ found:613.1691.
   G2(P6-C): ¹H NMR (400 MHz, CDCl₃) δ 8.30 (s, 1H), 7.51 (t, *J=* 8.5, 7.2 Hz, 1H), 7.12 (d, *J* = 7.1 Hz, 1H), 6.95 (d, *J=* 8.5 Hz, 1H), 4.94 (dd, *J=* 11.9, 5.4 Hz, 1H), 4.51 - 4.44 (m, 2H), 3.82 - 3.76 (m, 2H), 3.75 (t, *J=* 5.3 Hz, 2H), 3.69 (d, *J=* 3.7 Hz, 12H), 3.49 (t, *J* = 5.3 Hz, 2H), 2.93 - 2.87 (m, 1H), 2.86 (s, 4H), 2.81 - 2.72 (m, 2H), 2.24 - 2.07 (m, 1H). ¹³C NMR (101 MHz, CDCl₃) δ 171.06, 169.46 - 169.26 (m), 168.59, 168.29, 167.64, 151.64, 146.85, 136.02, 132.57, 129.65, 120.73, 116.79, 115.28, 111.64, 70.85, 70.71, 70.54, 70.53 - 70.50 (m), 70.28, 69.45, 68.31, 53.41, 48.89, 42.44, 31.42, 25.48, 22.83. ESI: [M + H]⁺ cacld: 679.2385, found: 679.2640. [M + NH₄]⁺ found: 696.2909. [M + Na]⁺ found: 701.2466.
5) Synthesis of a compound I: 2-(2,6-dioxopiperidin-3-yl)-4-fluoroindoline-1,3-dione (1 mmol, 1.0 equiv) was dissolved in N,N-dimethylacetamide (2 mL), and N-Boc-1,6-diaminohexane (1.2 mmol, 1.2 equiv) was added. The resulting reaction mixture was heated to 90°C and stirred for 2 h, and then subjected to extraction with ethyl acetate and water. The resulting crude product was purified by silica gel column chromatography to produce the compound I.
6) Synthesis of a compound J: The compound I (1 mmol, 1.0 equiv) was dissolved in dichloromethane (4 mL), and trifluoroacetic acid (1 mL) was added. The resulting mixture was stirred at room temperature for 6 h, and then concentrated to dryness under reduced pressure to produce the compound J.
7) Synthesis of compounds L: 6-hydroxyhexanoic acid (0.5 mmol, 1.2 equiv) or 10-hydroxydecanoic acid (0.5 mmol, 1.2 equiv) and 2-(7-azabenzotriazol-1-yl)-N,N,N',N'-tetramethyluronium hexafluorophosphate (HATU) (0.63 mmol, 1.5 equiv) were dissolved in N,N-dimethylformamide (2 mL), and then the compound J (0.42 mmol, 1.0 equiv) and N,N-diisopropylethylamine (1.26 mmol, 3 equiv) were added. The resulting mixture was stirred at room temperature for 6 h, subjected to extraction with ethyl acetate and water, dried over anhydrous sodium sulfate, and purified by silica gel column chromatography to produce the compounds L.
8) Synthesis of compounds M (NHS active esters of a small-molecule CRBN ligand that included linkers C12-1 and C16, respectively): A compound L (0.2 mmol, 1.0 equiv) and N,N'-disuccinimidyl carbonate (0.3 mmol, 1.5 equiv) were dissolved in anhydrous acetonitrile, and then triethylamine (0.6 mmol, 3.0 equiv) was added to produce a mixture. The mixture was stirred at room temperature for 16 h, subjected to solvent evaporation, subjected to extraction with dichloromethane and water, washed with a cold 5% sodium bicarbonate solution, dried over anhydrous sodium sulfate, and purified by silica gel column chromatography to produce the compounds M1 and M2.

M1(C12-C): ¹H NMR (400 MHz, CDCl₃) δ 8.46 (s, 1H), 7.50 (t, *J* = 8.5, 7.2 Hz, 1H), 7.09 (d, *J= 7.0* Hz, 1H), 6.89 (d, *J=* 8.5 Hz, 1H), 5.81 (s, 1H), 4.93 (dd, *J=* 11.9, 5.4 Hz, 1H), 4.33 (t, *J* = 6.4 Hz, 2H), 3.32 - 3.18 (m, 4H), 2.94 - 2.85 (m, 1H), 2.84 (s, 4H), 2.78 (m, 2H), 2.19 (t, *J* = 7.4 Hz, 2H), 2.16 - 2.09 (m, 1H), 1.82 - 1.73 (m, 2H), 1.73 - 1.62 (m, 5H), 1.55 - 1.50 (m, 2H), 1.48 - 1.34 (m, 6H). ¹³C NMR (101 MHz, CDCl₃) δ 172.79, 171.24, 169.54, 168.87, 168.55, 167.64, 151.58, 146.98, 136.16, 132.49, 116.69, 111.41, 109.87, 71.38, 48.89, 42.53, 39.39, 36.37, 31.41, 29.51, 29.07, 28.07, 26.57 , 25.49, 25.14, 22.80. [M + H]⁺ cacld: 628.2540, found: 628.2828. [M + Na]⁺ found: 650.2661.

M2(C16-C): ¹H NMR (400 MHz, CDCl₃) δ 8.42 (s, 1H), 7.50 (t, *J* = 8.4, 7.2 Hz, 1H), 7.09 (d, *J* = 7.1 Hz, 1H), 6.89 (d, *J=* 8.7 Hz, 1H), 5.80 (s, 1H), 4.92 (dd, *J=* 11.8, 5.4 Hz, 1H), 4.32 (t, *J* = 6.6 Hz, 2H), 3.27 (t, *J* = 7.0 Hz, 4H), 2.88 (m, 1H), 2.85 (s, 4H), 2.83 - 2.70 (m, 2H), 2.19 (t, *J* = 7.6 Hz, 2H), 2.15 - 2.10 (m, 1H), 1.79 - 1.70 (m, 2H), 1.70 - 1.59 (m, 4H), 1.57 - 1.50 (m, 2H), 1.44 (m, 2H), 1.39 (m, 4H), 1.30 (s, 8H). ¹³C NMR (101 MHz, CDCl₃) δ 173.47, 171.21, 169.54, 168.85, 168.51, 167.64, 151.62, 146.97, 136.16, 132.49, 129.51, 116.68, 115.39, 111.42, 109.88, 71.66, 48.89, 42.54, 39.41, 36.69, 31.41, 29.54, 29.16, 29.11, 28.93, 28.32, 26.59, 25.78, 25.49, 25.34, 22.80. [M + H]⁺ cacld: 684.3166, found: 684.3467. [M + Na]⁺ found: 706.3293.

### Example 3 Synthesis and identification of VHL ligand small molecules linked to linkers of different lengths

Synthetic routes were as follows: 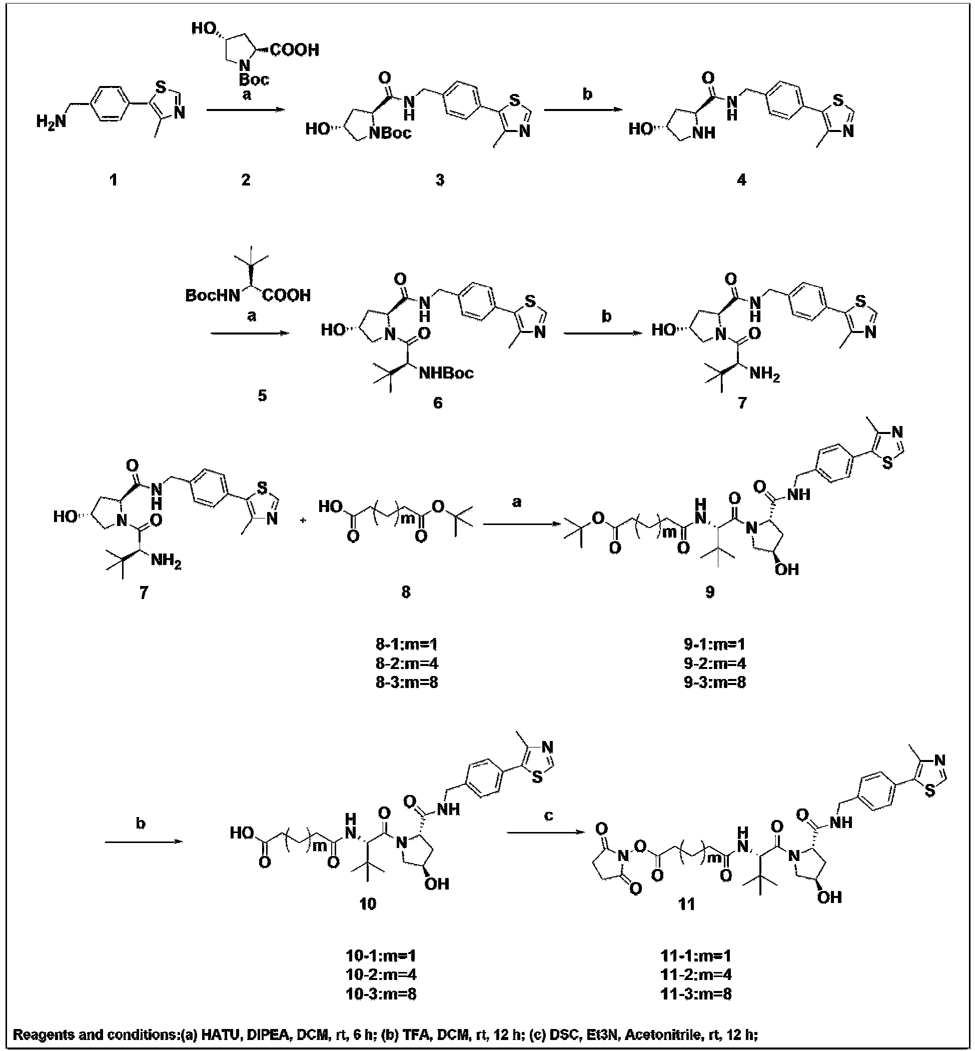
1) Synthesis of a compound 3: N-Boc-trans-4-hydroxy-L-proline methyl ester (1.2 mmol, 1.2 equiv) and HATU (1.5 mmol, 1.5 equiv) were dissolved in N,N-dimethylformamide (3 mL), and then 4-(4-methylthiazol-5-yl)benzylamine hydrochloride (1.0 mmol, 1.0 equiv) and N,N-diisopropylethylamine (3 mmol, 3 equiv) were added. The resulting mixture was stirred at room temperature for 12 h, subjected to extraction with ethyl acetate and water, dried over anhydrous sodium sulfate, and purified by silica gel column chromatography to produce the compound 3.
2) Synthesis of a compound 4: The compound 3 (0.84 mmol, 1.0 equiv) was dissolved in dichloromethane (4 mL), and trifluoroacetic acid (1 mL) was added. The resulting mixture was stirred at room temperature for 6 h, and then concentrated to dryness under reduced pressure to produce the compound 4.
3) Synthesis of a compound 6: N-Boc-L-tert-leucine (1 mmol, 1.2 equiv) and HATU (1.5 mmol, 1.5 equiv) were dissolved in N,N-dimethylformamide (3 mL), and then the compound 4 (0.84 mmol, 1.0 equiv) and N,N-diisopropylethylamine (3 mmol, 3 equiv) were added. The resulting mixture was stirred at room temperature for 12 h, subjected to extraction with ethyl acetate and water, dried over anhydrous sodium sulfate, and purified by silica gel column chromatography to produce the compound 6.
4) Synthesis of a compound 7: The compound 6 (0.84 mmol, 1.0 equiv) was dissolved in dichloromethane (4 mL), and trifluoroacetic acid (1 mL) was added. The resulting mixture was stirred at room temperature for 6 h, and then concentrated to dryness under reduced pressure to produce the compound 7.
5) Synthesis of a compound 9: A compound 8 (1 mmol, 1.2 equiv) and HATU (1.5 mmol, 1.5 equiv) were dissolved in N,N-dimethylformamide (3 mL), and then the compound 7 (0.84 mmol, 1.0 equiv) and N,N-diisopropylethylamine (3 mmol, 3 equiv) were added. The resulting mixture was stirred at room temperature for 12 h, subjected to extraction with ethyl acetate and water, dried over anhydrous sodium sulfate, and purified by silica gel column chromatography to produce the compound 9.
6) Synthesis of a compound 10: The compound 9 (0.84 mmol, 1.0 equiv) was dissolved in dichloromethane (4 mL), and trifluoroacetic acid (1 mL) was added. The resulting mixture was stirred at room temperature for 6 h, and then concentrated to dryness under reduced pressure to produce the compound 10.
7) Synthesis of compounds 11 (NHS active esters of a VHL ligand that included linkers C5, C8, and C12-2, respectively): The compound 10 (0.09 mmol, 1.0 equiv) and NHS (0.14 mmol, 1.5 equiv) were dissolved in dichloromethane (2 mL), and then 1-ethyl-3-(3-dimethylaminopropyl)carbodiimide (0.16 mmol, 1.8 equiv) was added. The resulting mixture was stirred at room temperature for 12 h, washed with water and saturated brine, dried over anhydrous sodium sulfate, and purified by column chromatography to produce a compound 11-1, a compound 11-2, and a compound 11-3.

11-1 (C5-V): ¹H NMR (400 MHz, CD₃CN) δ 8.77 (s, 1H), 7.48 - 7.39 (m, 4H), 7.28 (s, 1H), 6.75 (d, *J=* 8.8 Hz, 1H), 4.58 (d, *J=* 8.9 Hz, 1H), 4.54 - 4.42 (m, 3H), 4.33 (dd, *J* = 15.5, 5.7 Hz, 1H), 3.86 (d, *J* = 11.0 Hz, 1H), 3.70 (d, *J* = 11.0 Hz, 1H), 3.44 (s, 1H), 2.79 (s, 4H), 2.49 (s, 3H), 2.41 - 2.29 (m, 2H), 2.25 (s, 4H), 2.15 (s, 2H), 0.98 (s, 9H). ¹³C NMR (101 MHz, CD₃CN) δ 171.82, 171.70, 170.83, 170.26, 168.88, 150.74, 148.37, 139.30, 130.47, 129.10, 127.70, 69.80, 59.09, 57.22, 56.56, 54.23, 42.18, 37.36, 35.02, 33.76, 29.74, 28.74, 25.76, 25.41, 20.66, 15.43. [M + H]⁺ cacld: 642.2519, found: 642.2651. [M + Na]⁺ found: 664.2474.

11-2 (C8-V): ¹H NMR (400 MHz, CDCl₃) δ 8.73 (s, 1H), 7.41 - 7.30 (m, 4H), 6.34 (d, *J* = 8.9 Hz, 1H), 4.69 (t, *J* = 8.1 Hz, 1H), 4.62 - 4.51 (m, 3H), 4.36 (dd, *J=* 15.0, 5.3 Hz, 1H), 4.11 (d, *J* = 11.4 Hz, 1H), 3.64 (dd, *J* = 11.4, 3.4 Hz, 1H), 2.80 (s, 4H), 2.59 (t, *J* = 7.2 Hz, 2H), 2.54 (s, 3H), 2.51 - 2.41 (m, 1H), 2.30 - 2.22 (m, 2H), 2.17 (dd, *J=* 13.5, 8.0 Hz, 1H), 1.79 - 1.69 (m, 2H), 1.68 - 1.58 (m, 2H), 1.46 - 1.38 (m, 2H), 1.37 - 1.27 (m, 3H), 0.97 (s, 9H). ¹³C NMR (101 MHz, CDCl₃) δ 173.98, 171.79, 171.04, 169.55, 168.74, 156.13, 150.52, 138.20, 130.81, 129.59, 128.15, 120.31, 115.39, 70.10, 58.68, 57.50, 56.86, 43.27, 36.15, 35.84, 34.99, 30.79, 28.03, 27.90, 26.39, 25.61, 25.01, 24.37, 15.90. [M + H]⁺ cacld: 684.2989, found: 684.2056. [M + Na]⁺ found: 706.2908.

11-3 (C12-V): ¹H NMR (400 MHz, CDCl₃) δ 8.86 (s, 1H), 7.39 (s, 4H), 6.12 (d, J=8.7 Hz, 1H), 4.74 (t, *J*=7.9 Hz, 1H), 4.68-4.48 (m, 3H), 4.37 (dd, *J*=15.0, 5.2 Hz, 1H), 4.14 (d, *J*=11.3 Hz, 1H), 3.62 (dd, *J*=11.4, 3.6 Hz, 2H), 2.85 (s, 4H), 2.61 (t, J=7.5 Hz, 2H), 2.58 (s, 3H), 2.57-2.53 (m, 1H), 2.21 (t, J=7.6 Hz, 2H), 2.17-2.08 (m, 1H), 1.81-1.71 (m, 2H), 1.61 (s, 2H), 1.40 (t, *J*=7.8 Hz, 2H), 1.29 (s, 10H), 0.96 (s, 9H). ¹³C NMR (101 MHz, CDCl₃) δ 174.05, 171.78, 171.01, 169.38, 168.76, 156.39, 150.62, 138.19, 129.57, 128.12, 120.08, 115.42, 70.03, 58.79, 57.45, 56.85, 43.23, 36.52, 36.10, 35.14, 30.91, 29.27, 29.19, 28.97, 28.70, 26.41, 25.61, 24.52, 15.89. [M + H]⁺ cacld: 740.3615, found: 740.3734. [M + Na]⁺ found: 762.3556.

### Example 4 Coupling of nucleic acids to small-molecule CRBN/VHL ligands linked to linkers of different lengths, characterization of coupled products, and influence of the coupled products on Lin28A/B expression levels

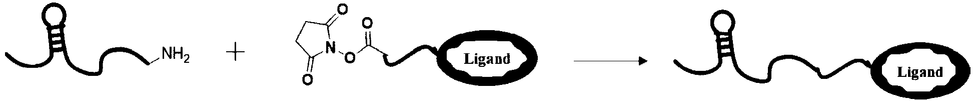

An oligonucleotide sequence ORN-C7-NH₂ including C7-NH₂ at a 3' terminus was synthesized using a solid-phase synthesizer. Then, ORN-C7-NH₂ (10.0 nmol, 1.0 equiv) was dissolved in 50 µL of 1×PBS to produce an oligonucleotide sequence solution. The NHS active esters of small-molecule CRBN/VHL ligands that included linkers of different lengths (5.0 µmol, 500 equiv) synthesized in Examples 2 and 3 were each dissolved in 50 µL of DMSO to produce NHS active ester solutions. The oligonucleotide sequence solution and each NHS active ester solution were then mixed and shaken at room temperature for 12 h, subjected to dialysis using a 1,000 D dialysis bag, and purified by high-performance liquid chromatography (HPLC) to produce oligonucleotide-based PROTAC molecules. Structural compositions, mass spectrometry characterization results, and Lin28A/B expression levels at a concentration of 100 nM for 48 h for the PROTAC molecules were shown in Tables 2 and 3.

**Table 2 Structural compositions and mass spectrometry characterization results for the PROTAC molecules**

| PROTAC molecule | Oligonucleotide sequence capable of binding to Lin28A/B | Linker | CRBN/ VHL ligand | Actual molecular weight | Lin28A (T47D) expression level | Lin28B (H1299) expression level |
|---|---|---|---|---|---|---|
| Let-7a-1 Full-S P4-C | UUAGGGUCACACCCACCACUGGGAGAU | P4 | CRBN | 9734.2 | 39% | 100% |
| Let-7a-2 Full-S P4-C | UAGAAUUACAUCAAGGGAGAU | P4 | CRBN | 7775.2 | 39% | 98% |
| Let-7a-3 Full-S P4-C | UGGGGCUCUGCCCUGCUAUGGGAU | P4 | CRBN | 8733.2 | 99% | 100% |
| Let-7b Full-S P4-C | UCAGGGCAGUGAUGUUGCCCCUCGGAAGAU | P4 | CRBN | 10796.8 | 13% | 47% |
| Let-7c Full-S P4-C | UAGAGUUACACCCUGGGAGUU | P4 | CRBN | 7719.5 | 12% | 100% |
| Let-7d Full-S P4-C | UUAGGGCAGGGAUUUUGCCCACAAGGAGGU | P4 | CRBN | 10860.1 | 35% | 55% |
| Let-7e Full-S P4-C | GAGGAGGACACCCAAGGAGAU | P4 | CRBN | 7866.8 | 72% | 100% |
| Let-7f-1 Full-S P4-C | GUGGGGUAGUGAUUUUACCCUGUUCAGGAGAU | P4 | CRBN | 11482.0 | 24% | 40% |
| Let-7f-2 Full-S P4-C | UUAGGGUCAUACCCCAUCUUGGAGAU | P4 | CRBN | 9352.5 | 25% | 100% |
| Let-7g Full-S P4-C | UGAGGGUCUAUGAUACCACCCGGUACAGGAGAU | P4 | CRBN | 11832.6 | 26% | 64% |
| Let-7i Full-S P4-C | GGUCGGGUUGUGACAUUGCCCGCUGUGGAGAU | P4 | CRBN | 11512.7 | 26% | 62% |
| miR98 Full-S P4-C | GUGGGGUAGGGAUAUUAGGCCCCAAUUAGAAGAU | P4 | CRBN | 12258.7 | 32% | 94% |
| Let-7f-1 Full-S C3-C | GGGGUAGUGAUUUUACCCUGUUCAGGAGAU | C3 | CRBN | 10696.3 | 31% | 100% |
| Let-7f-1 Full-S C6-C | GGGGUAGUGAUUUUACCCUGUUCAGGAGAU | C6 | CRBN | 10737.4 | 30% | 94% |
| Let-7f-1 Full-S C12-C | GGGGUAGUGAUUUUACCCUGUUCAGGAGAU | C12-1 | CRBN | 10849.5 | 22% | 97% |
| Let-7f-1 Full-S C16-C | GGGGUAGUGAUUUUACCCUGUUCAGGAGAU | C16 | CRBN | 10904.9 | 29% | 91% |
| Let-7f-1 Full-S P4-C | GGGGUAGUGAUUUUACCCUGUUCAGGAGAU | P4 | CRBN | 10814.4 | 24% | 40% |
| Let-7f-1 Full-S P6-C | GGGGUAGUGAUUUUACCCUGUUCAGGAGAU | P6 | CRBN | 10901.6 | 25% | 79% |
| Let-7f-1 Full-S C5-V | GGGGUAGUGAUUUUACCCUGUUCAGGAGAU | C5 | VHL | 10863.7 | 46% | 90% |
| Let-7f-1 Full-S C8-V | GGGGUAGUGAUUUUACCCUGUUCAGGAGAU | C8 | VHL | 10986.2 | 84% | 85% |
| Let-7f-1 Full-S C12-V | GGGGUAGUGAUUUUACCCUGUUCAGGAGAU | C12-2 | VHL | 10962.1 | 20% | 76% |
| Let-7f-1 CSD-S C3-C | GGGGUAGUGAUUUUACCCUGUUCA | C3 | CRBN | 8584.0 | 21% | 100% |
| Let-7f-1 CSD-S C6-C | GGGGUAGUGAUUUUACCCUGUUCA | C6 | CRBN | 8625.2 | 16% | 100% |
| Let-7f-1 CSD-S C12-C | GGGGUAGUGAUUUUACCCUGUUCA | C12-1 | CRBN | 8738.4 | 63% | 100% |
| Let-7f-1 CSD-S C16-C | GGGGUAGUGAUUUUACCCUGUUCA | C16 | CRBN | 8795.5 | 20% | 100% |
| Let-7f-1 CSD-S P4-C | GGGGUAGUGAUUUUACCCUGUUCA | P4 | CRBN | 8701.2 | 93% | 100% |
| Let-7f-1 CSD-S P6-C | GGGGUAGUGAUUUUACCCUGUUCA | P6 | CRBN | 8789.1 | 71% | 100% |
| Let-7f-1 CSD-S C5-V | GGGGUAGUGAUUUUACCCUGUUCA | C5 | VHL | 8752.2 | 63% | 86% |
| Let-7f-1 CSD-S C8-V | GGGGUAGUGAUUUUACCCUGUUCA | C8 | VHL | 8794.8 | 84% | 94% |
| Let-7f-1 CSD-S C12-V | GGGGUAGUGAUUUUACCCUGUUCA | C12-2 | VHL | 8850.6 | 12% | 88% |
| Let-7f-1 ZKD -S C3-C | AGGAGAU | C3 | CRBN | 2929.9 | 90% | 96% |
| Let-7f-1 ZKD-S C6-C | AGGAGAU | C6 | CRBN | 2971.5 | 100% | 94% |
| Let-7f-1 ZKD-S C12-C | AGGAGAU | C12-1 | CRBN | 3085.5 | 90% | 96% |
| Let-7f-1 ZKD-S C16-C | AGGAGAU | C16 | CRBN | 3141.1 | 73% | 78% |
| Let-7f-1 ZKD-S P4-C | AGGAGAU | P4 | CRBN | 3048.7 | 85% | 99% |
| Let-7f-1 ZKD-S P6-C | AGGAGAU | P6 | CRBN | 3135.9 | 94% | 100% |
| Let-7f-1 ZKD-S C5-V | AGGAGAU | C5 | VHL | 3099.6 | 100% | 93% |
| Let-7f-1 ZKD-S C8-V | AGGAGAU | C8 | VHL | 3141.8 | 96% | 100% |
| Let-7f-1ZKD-S C12-V | AGGAGAU | C12-2 | VHL | 3197.3 | 90% | 84% |

**Table 3 Structural compositions and mass spectrometry characterization results for the PROTAC molecules**

| PROTAC molecule | Oligonucleotide sequence capable of binding to Lin28A/B | Linker | CRBN/ VHL ligand | Actual molecular weight | Lin28B (Huh7) expression level |
|---|---|---|---|---|---|
| Let-7a-1 Full-S P4-C | UUAGGGUCACACCCACCACUGGGAGAU | P4 | CRBN | 9734.2 | 70% |
| Let-7a-2 Full-S P4-C | UAGAAUUACAUCAAGGGAGAU | P4 | CRBN | 7775.2 | 31% |
| Let-7a-3 Full-S P4-C | UGGGGCUCUGCCCUGCUAUGGGAU | P4 | CRBN | 8733.2 | 56% |
| Let-7b Full-S P4-C | UCAGGGCAGUGAUGUUGCCCCUCGGAAGAU | P4 | CRBN | 10796.8 | 12% |
| Let-7c Full-S P4-C | UAGAGUUACACCCUGGGAGUU | P4 | CRBN | 7719.5 | 48% |
| Let-7d Full-S P4-C | UUAGGGCAGGGAUUUUGCCCACAAGGAGGU | P4 | CRBN | 10860.1 | 25% |
| Let-7e Full-S P4-C | GAGGAGGACACCCAAGGAGAU | P4 | CRBN | 7866.8 | 59% |
| Let-7f-1 Full-S P4-C | GUGGGGUAGUGAUUUUACCCUGUUCAGGAGAU | P4 | CRBN | 11482.0 | 12% |
| Let-7f-2 Full-S P4-C | UUAGGGUCAUACCCCAUCUUGGAGAU | P4 | CRBN | 9352.5 | 50% |
| Let-7g Full-S P4-C | UGAGGGUCUAUGAUACCACCCGGUACAGGAGAU | P4 | CRBN | 11832.6 | 30% |
| Let-7i Full-S P4-C | GGUCGGGUUGUGACAUUGCCCGCUGUGGAGAU | P4 | CRBN | 11512.7 | 37% |
| miR98 Full-S P4-C | GUGGGGUAGGGAUAUUAGGCCCCAAUUAGAAGAU | P4 | CRBN | 12258.7 | 36% |
| Let-7b Full-S C3-C | UCAGGGCAGUGAUGUUGCCCCUCGGAAGAU | C3 | CRBN | 10679.3 | 12% |
| Let-7b Full-S C6-C | UCAGGGCAGUGAUGUUGCCCCUCGGAAGAU | C6 | CRBN | 10722.2 | 19% |
| Let-7b Full-S C12-C | UCAGGGCAGUGAUGUUGCCCCUCGGAAGAU | C12-1 | CRBN | 10834.7 | 10% |
| Let-7b Full-S C16-C | UCAGGGCAGUGAUGUUGCCCCUCGGAAGAU | C16 | CRBN | 10891.3 | 13% |
| Let-7b Full-S P4-C | UCAGGGCAGUGAUGUUGCCCCUCGGAAGAU | P4 | CRBN | 10796.8 | 10% |
| Let-7b Full-S P6-C | UCAGGGCAGUGAUGUUGCCCCUCGGAAGAU | P6 | CRBN | 10885.7 | 9% |
| Let-7b Full-S C5-V | UCAGGGCAGUGAUGUUGCCCCUCGGAAGAU | C5 | VHL | 10848.2 | 30% |
| Let-7b Full-S C8-V | UCAGGGCAGUGAUGUUGCCCCUCGGAAGAU | C8 | VHL | 10890.4 | 15% |
| Let-7b Full-S C12-V | UCAGGGCAGUGAUGUUGCCCCUCGGAAGAU | C12-2 | VHL | 10947.7 | 17% |
| Let-7b CSD-S C3-C | UCAGGGCAGUGAUGUUGCCCCUCG | C3 | CRBN | 8597.6 | 22% |
| Let-7b CSD-S C6-C | UCAGGGCAGUGAUGUUGCCCCUCG | C6 | CRBN | 8640.2 | 15% |
| Let-7b CSD-S C12-C | UCAGGGCAGUGAUGUUGCCCCUCG | C12-1 | CRBN | 8753.7 | 11% |
| Let-7b CSD-S C16-C | UCAGGGCAGUGAUGUUGCCCCUCG | C16 | CRBN | 8809.2 | 27% |
| Let-7b CSD-S P4-C | UCAGGGCAGUGAUGUUGCCCCUCG | P4 | CRBN | 8716.3 | 21% |
| Let-7b CSD-S P6-C | UCAGGGCAGUGAUGUUGCCCCUCG | P6 | CRBN | 8804.5 | 15% |
| Let-7b CSD-S C5-V | UCAGGGCAGUGAUGUUGCCCCUCG | C5 | VHL | 8767.9 | 59% |
| Let-7b CSD-S C8-V | UCAGGGCAGUGAUGUUGCCCCUCG | C8 | VHL | 8810.2 | 67% |
| Let-7b CSD-S C12-V | UCAGGGCAGUGAUGUUGCCCCUCG | C12-2 | VHL | 8865.7 | 59% |
| Let-7b ZKD -S C3-C | GGAAGAU | C3 | CRBN | 2930.3 | 99% |
| Let-7b ZKD-S C6-C | GGAAGAU | C6 | CRBN | 2972.3 | 99% |
| Let-7b ZKD-S C12-C | GGAAGAU | C12-1 | CRBN | 3085.8 | 95% |
| Let-7b ZKD-S C16-C | GGAAGAU | C16 | CRBN | 3142.3 | 92% |
| Let-7b ZKD-S P4-C | GGAAGAU | P4 | CRBN | 3049.2 | 100% |
| Let-7b ZKD-S P6-C | GGAAGAU | P6 | CRBN | 3136.5 | 95% |
| Let-7b ZKD-S C5-V | GGAAGAU | C5 | VHL | 3099.8 | 100% |
| Let-7b ZKD-S C8-V | GGAAGAU | C8 | VHL | 3142.5 | 89% |
| Let-7b ZKD-S C12-V | GGAAGAU | C12-2 | VHL | 3198.0 | 100% |

### Experimental Example 1 Targeted degradation of a Lin28A protein and subsequent bioactivity evaluation experiments

### 1. Experiment for evaluating an effect of targeted degradation of Lin28A

### 1.1 Experimental method

Cell culture and transfection: For the target protein Lin28A, T47D (human breast ductal carcinoma) cells were selected for cell-based activity evaluation. Cells were inoculated into a 12-well plate at 2 × 10⁵ cells/well to achieve a cell density of 70% to 80%, and then cultured overnight in a 37°C and 5% CO₂ incubator. Transfection was conducted with Lipofectamine 2000. According to instructions, Lipofectamine 2000 was diluted with an OptiMEM medium and incubated for 5 min. Lin28-targeting PROTAC molecules and a control molecule were each taken at an amount enabling 100 nM/well and diluted with Opti-MEM. The resulting Lipofectamine 2000 solution was added to each of resulting Lin28-PROTAC molecule and control molecule solutions, and thorough mixing was achieved by pipetting up and down. Incubation was conducted for 10 min to produce transfection complexes. An original medium in each well of the 12-well plate was replaced with a fresh medium, and the transfection complexes were added. Incubation was conducted for 48 h.

For a time-dependent effect, a transfection concentration for the Lin28-targeting PROTAC molecules and the control molecule was 100 nM, and transfection times were 12 h, 24 h, 48 h, and 72 h. For a concentration-dependent effect, transfection concentrations for the Lin28-targeting PROTAC molecules and the control molecule were 25 nM, 50 nM, 100 nM, and 200 nM, and a transfection time was 48 h.

Detection of Lin28A expression levels: After being cultured for an appropriate time, cells were digested, collected through centrifugation, and subjected to lysis with a radio-immunoprecipitation assay (RIPA) lysis buffer for 30 min. Centrifugation was conducted at 12,000 rpm and 4°C for 15 min. The resulting protein was quantified according to instructions of a bicinchoninic acid (BCA) kit, and prepared into a protein solution with a desired concentration. A sodium dodecyl sulfate-polyacrylamide gel electrophoresis (SDS-PAGE) loading buffer was added. The resulting mixed solution was boiled at 100°C for 5 min and stored at -20°C.

An SDS-PAGE gel of an appropriate concentration was prepared. A prepared protein sample was centrifuged, thoroughly mixed, and loaded into wells of the SDS-PAGE gel. Electrophoresis was conducted at a voltage of 90 V for 15 min until the protein sample entered a separating gel. Subsequently, the voltage was adjusted to 120 V, and the electrophoresis was continued for an appropriate period of time and then terminated. After the electrophoresis was completed, the SDS-PAGE gel was collected. Proteins were transferred onto a nitrocellulose (NC) membrane at a constant current of 300 mA for 60 min on ice. The NC membrane was collected, blocked with 5% non-fat dry milk, and washed three times with TBST. A primary antibody diluted at an appropriate dilution ratio was added, and incubation was conducted overnight at 4°C. Washing was conducted three times with TBST, where shaking was conducted for 5 min each time. The TBST was discarded, and a diluted secondary antibody was added. Incubation was conducted for 1 h on a shaker at room temperature. The diluted secondary antibody was discarded. Washing was conducted three times with TBST, where shaking was conducted for 5 min each time. Finally, visualization and imaging were conducted using an Odyssey two-color infrared laser imaging system.

Ubiquitination effect evaluation: 5 × 10⁵ cells were inoculated into each 6 cm petri dish, cultured for 12 h until cell adhesion was achieved, then transfected with a His-ub plasmid, further cultured for 24 h, then transfected with Full-S ORN and Full-S P4-C samples, and further cultured for 24 h. 4 h before cell harvesting, MG132 was added at 20 µM. Cells were digested, collected through centrifugation, and washed with 1× PBS, and then 500 µL of a denaturing lysis buffer B (including 8 M urea, 100 mM NaH₂PO₄, 10 mM Tris, and 25 mM imidazole, pH 8.0) was added. Lysis was conducted on ice for 15 min, and then ultrasonic lysis was conducted using an ultrasonic cell disruptor. After the lysis was completed, centrifugation was conducted at 12,000 rpm and 4°C for 15 min. The resulting supernatant was collected and analyzed using the BCA kit.

150 µL of nickel beads were taken and washed twice with PBS, where centrifugation was conducted at 550 g and 4°C for 3 min and the resulting supernatant was discarded each time. Washed nickel beads were then washed twice with a denaturing lysis buffer B, where during each time, 150 µL of the denaturing lysis buffer B was added and centrifugation was conducted at 550 g and 4°C for 3 min. Aliquots of nickel beads were added to quantified protein samples, and incubation was conducted on a shaker at 4°C for 4 h to 6 h. After the incubation was completed, centrifugation was conducted at 550 g and 4°C for 4 min, and the resulting supernatant was discarded. Washing was conducted three times with a lysis buffer C (including 8 M urea, 100 mM NaH₂PO₄, 10 mM Tris, and 25 mM imidazole, pH 6.3), where centrifugation was conducted at 550 g and 4°C for 3 min each time. Finally, elution was conducted twice, where during each time, 25 µL of a solution E (including 8 M urea, 100 mM NaH₂PO₄, 10 mM Tris, and 250 mM imidazole, pH 4) was added and centrifugation was conducted at 550 g and 4°C for 10 min. Then, 10 µL of 5× loading buffer was added, and boiling was conducted at 100°C for 5 min. Western Blot (WB) analysis was conducted according to the experimental procedure described in the section 1.1.2.

### 2. Experimental results

### Evaluation of degradation effects of Lin28A in T47D cells:

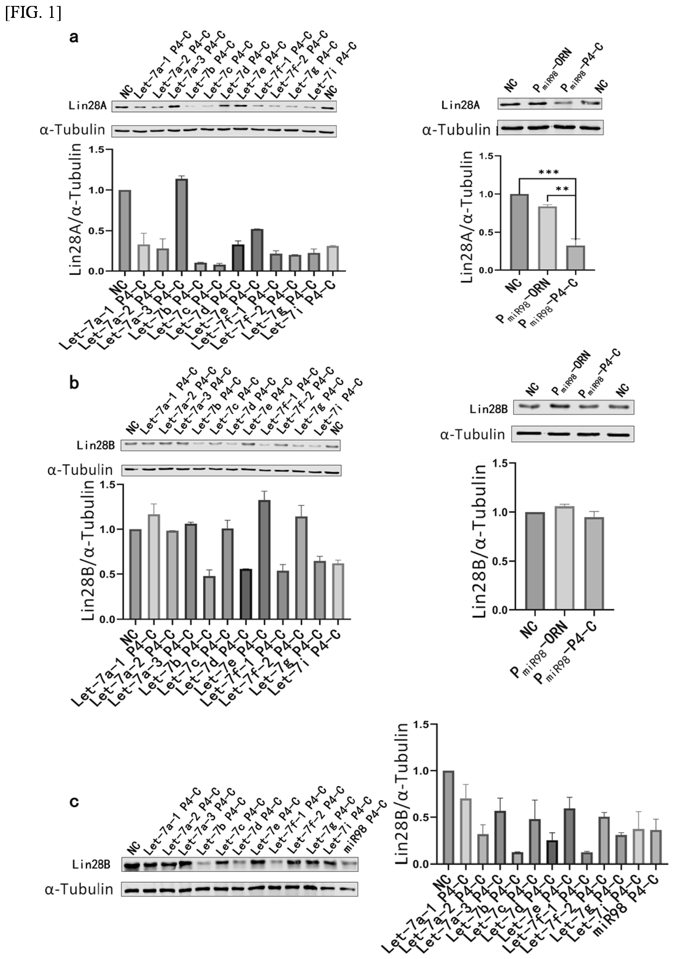

After being coupled to pomalidomide through a PEG4 linker, all sequences capable of binding to the Lin28A protein exhibited significant degradation activity except for Let-7a-3 P4-C (Let-7a-3 Full-S P4-C) and Let-7e P4-C (Let-7e Full-S P4-C). Let-7b P4-C (Let-7b Full-S P4-C), Let-7c P4-C (Let-7c Full-S P4-C), and Let-7f-1 P4-C (Let-7f-1 Full-S P4-C) exhibited the most pronounced effect. The Let-7f-1 sequence (SEQ ID No. 13) was selected for subsequent screening of linker lengths and E3 Ub ligase ligands (FIG. 1a).

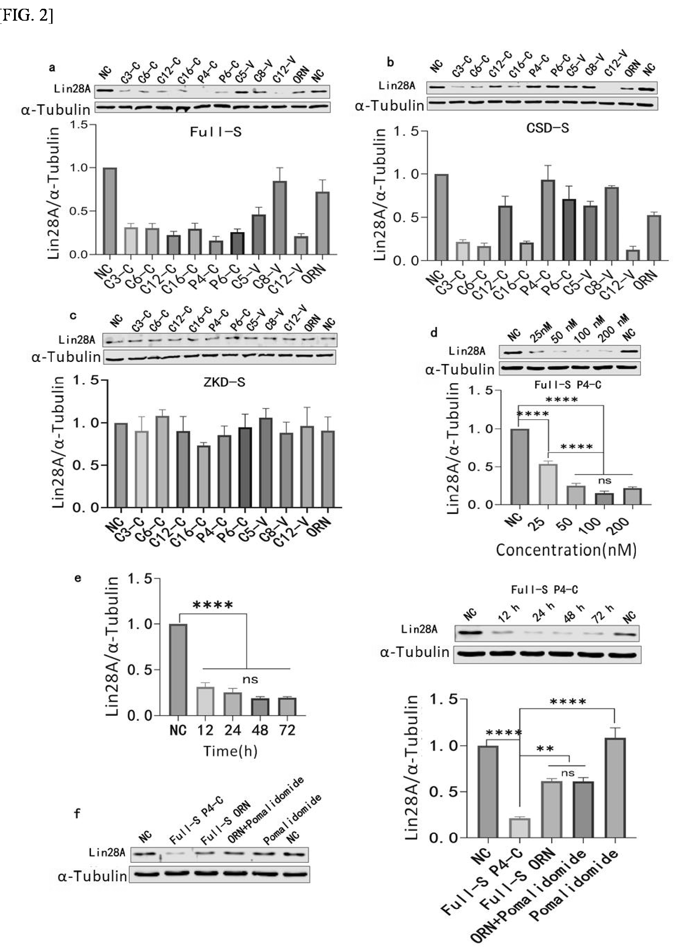

Under conditions of 48 h and 100 nM, all samples produced from Full-S sequences capable of binding to both CSD and ZKD except for Full-S C8-V (Let-7f-1 Full-S C8-V) exhibited significant degradation activity against the Lin28A protein. Full-S P4-C (Let-7f-1 Full-S P4-C) and Full-S C12-V (Let-7f-1 Full-S C12-V) showed the optimal activity (FIG. 2a). Among CSD-S sequences capable of binding only to CSD, CSD-S C3-C (Let-7f-1 CSD-S C3-C), CSD-S C6-C (Let-7f-1 CSD-S C6-C), CSD-S C16-C (Let-7f-1 CSD-S C16-C), and CSD-S C12-V (Let-7f-1 CSD-S C12-V) exhibited significant degradation activity against the Lin28A protein (FIG. 2b). All samples produced from ZKD-S sequences capable of binding only to ZKD did not show significant degradation activity against the Lin28A protein (FIG. 2c). The Full-S P4-C sample (Let-7f-1 Full-S P4-C) was then selected for evaluation of time-dependent and concentration-dependent effects on Lin28A protein degradation. Under the condition of 48 h, Full-S P4-C exhibited a significant concentration-dependent effect, and achieved a maximum degradation effect at 50 nM (FIG. 2d). At a transfection concentration of 100 nM, Full-S P4-C achieved a maximum degradation effect at 12 h (FIG. 2e). A Full-S P4-C experimental group exhibited a better degradation effect on Lin28A than a Full-S ORN experimental group without coupled pomalidomide and a Full-ORN/pomalidomide mixture group. Moreover, an experimental group treated with pomalidomide alone did not lead to significant degradation of the Lin28A protein. It indicated that the degradation of Lin28A mediated by Full-S P4-C was specific degradation mediated jointly by Full-S ORN and pomalidomide that were coupled, rather than non-specific degradation mediated by any single one of the aforementioned components alone (FIG. 2f). According to a ubiquitination experiment, a Full-S P4-C treatment increased the polyubiquitination of the Lin28A protein in T47D cells. The addition of the proteasome inhibitor MG132 substantially attenuated the degradation effect of Full-S P4-C on Lin28A, indicating that Full-S P4-C mediated the degradation of Lin28A through a proteasome pathway.

### 2. Experiment on miRNA let-7 level changes after Full-S P4-C-mediated degradation of Lin28A

### 2.1 Experimental method

Cell culture and transfection: T47D cells were inoculated into a 24-well plate at 1 × 10⁵ cells/well to achieve a cell density of 70% to 80%, and then cultured overnight in a 37°C and 5% CO₂ incubator. Transfection was conducted as described in the section 1.1.

### Detection of miRNA let-7 expression levels:

Extraction of miRNA: Cells were lysed with a lysis buffer MZ for 5 min, then transferred to a 1.5 mL centrifuge tube, and allowed to stand at room temperature for 5 min. Then, 40 µL of chloroform was added, and vigorous shaking was conducted for 15 s. The centrifuge tube was allowed to stand at room temperature for 5 min and then centrifuged at 12,000 rpm and 4°C for 15 min. The resulting uppermost aqueous phase was transferred to a new centrifuge tube, absolute ethanol was slowly added in a volume 0.43-fold a volume of the uppermost aqueous phase, and thorough mixing was conducted. The resulting mixture was transferred into an adsorption column miRspin and centrifuged at 12,000 rpm and 4°C for 30 s. An effluent was collected, absolute ethanol was slowly added in a volume 0.75-fold a volume of the effluent, and thorough mixing was conducted. The resulting mixture was transferred into an adsorption column miRelute and centrifuged at 12,000 rpm and 4°C for 30 s. An effluent was discarded. The adsorption column miRelute was retained. 500 µL of a protein removal solution MRD was added to the adsorption column miRelute. The adsorption column miRelute was then allowed to stand at room temperature for 2 min and centrifuged at 12,000 rpm and 4°C for 30 s. The resulting waste liquid was discarded. 500 µL of a wash solution RW was added to the adsorption column miRelute. The adsorption column miRelute was allowed to stand at room temperature for 2 min and centrifuged at 12,000 rpm and 4°C for 30 s. The resulting waste liquid was discarded. The washing operation was repeated twice. The adsorption column miRelute was placed into a 2 mL collection tube, centrifuged at 12,000 rpm and 4°C for 1 min to achieve residual liquid removal, and then allowed to stand at room temperature for a short period. The adsorption column miRelute was transferred to a new 1.5 mL centrifuge tube, and 15 µL to 30 µL of RNase-Free ddH₂O was added. The centrifuge tube was allowed to stand at room temperature for 2 min and then centrifuged at 12,000 rpm and 4°C for 2 min.

Reverse transcription experiment: The extracted miRNA was quantified and then prepared into a 40 ng/µL solution. The miRNA and reverse transcription reagents (Total RNA: 8 µL; 2× miRNA RT Reaction Buffer: 10 µL; and miRNA RT Enzyme Mix: 2 µL) were added to a RNase-free centrifuge tube. Reverse transcription was conducted according to a reverse transcription program (42°C for 60 min and 95°C for 3 min).

Reverse transcription-polymerase chain reaction (RT-PCR) detection: Primers used for the RT-PCR detection were shown in Table 4. A RNase-free centrifuge tube was pre-cooled on ice, and then cDNA and RT-PCR reagents (2x miRcute Plus miRNA PreMix: 10 µL; 2 µM Forward Primer: 2 µL; 2 µM Reverse Primer: 2 µL; and first-strand cDNA of miRNA: 6 µL) were added. An RT-PCR program was as follows: 1 cycle of 95°C for 15 min; and 40 cycles of 94°C for 20 s and 60°C for 34 s.

**Table 4 Primers for RT-PCR detection**

| Gene | Forward primer | Reverse primer |
|---|---|---|
| Let-7a | GCGTGAGGTAGTAGGTTGTATAGTTAAA | |
| Let-7b | GCGTGAGGTAGTAGGTTGTGTGGTTAAA | |
| Let-7c | GCGTGAGGTAGTAGGTTGTATGGTTAAA | |
| Let-7d | GCGAGAGGTAGTAGGTTGCATAGTTAAA | |
| Let-7e | GCGTGAGGTAGGAGGTTGTATAGTTAAA | Universal primer included in a kit |
| Let-7f | GCGTGAGGTAGTAGATTGTATAGTTAAA | |
| Let-7g | GCGTGAGGTAGTAGTTTGTACAGTTAAA | |
| Let-7i | GCGTGAGGTAGTAGTTTGTGCTGTTAAA | |
| miR-21 | GCGUAGCUUAUCAGACUGAUGUUGAAAA | |
| U6 | CTCGCTTCGGCAGCACA | AACGCTTCACGAATTTGCG |

### 2.2 Experimental results

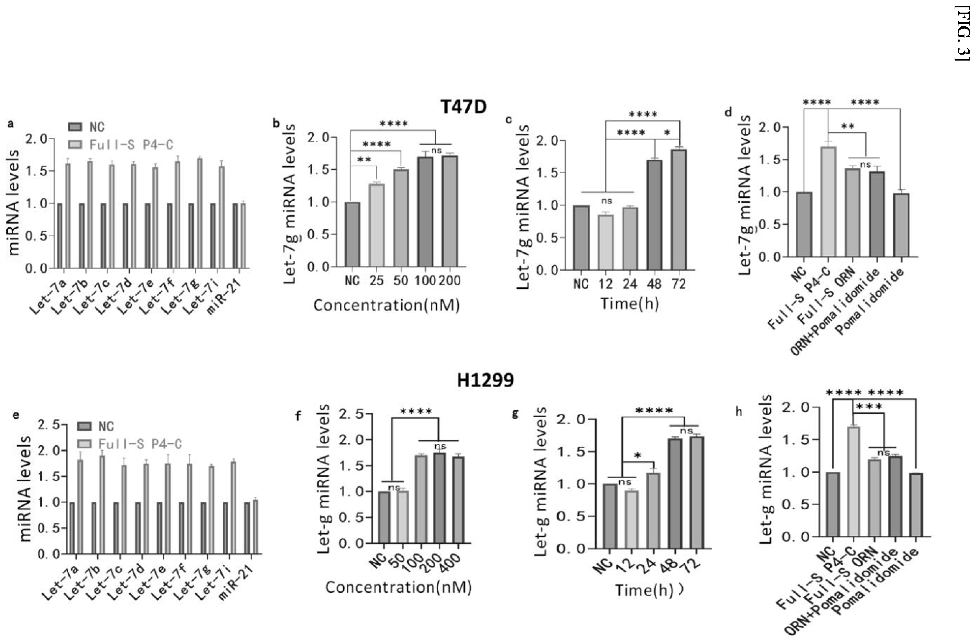

miRNA let-7 expression levels were shown in FIG. 3. As the Lin28A protein was degraded, the expression levels of all mature let-7 members were upregulated by 1.5-fold or more, and the expression level of miR-21 remained unchanged. It indicated that the targeted degradation of Lin28A by Full-S P4-C specifically led to an increase in let-7 expression levels (FIG. 3a). Under the condition of 48 h, as the concentration of Full-S P4-C increased, the expression level of let-7g was gradually upregulated, and reached a maximum value at 100 nM (FIG. 3b). At a transfection concentration of 100 nM, as the incubation time extended, the expression level of let-7g markedly increased at 48 h, and increased slightly with the further prolongation of time (FIG. 3c). Under the same transfection concentration and time, the Full-S P4-C experimental group exhibited a better effect on increasing let-7g levels than the Full-S ORN experimental group without coupled pomalidomide and the Full-ORN/pomalidomide mixture group, while the experimental group treated with pomalidomide alone did not show a significant increase in let-7g levels (FIG. 3d).

### 3. Experiment on the inhibition of T47D cell proliferation by Full-S P4-C in combination with tamoxifen

### 3.1 Experimental method

MTT assay procedure: Cells were inoculated into a 96-well plate at 1.5 × 10⁴ cells/well (100 µL) and cultured overnight in a 37°C and 5% CO₂ incubator. Transfection was conducted as described above. At 24 h, 48 h, and 72 h of cell culturing, 10 µL of an MTT solution (5 mg/mL) was added to each well. Cells were further cultured for 4 h. The resulting culture supernatant in each well was carefully discarded. 150 µL of DMSO was added to each well, and shaking was conducted for 10 min. An absorbance value of each well was measured at a wavelength of 490 nm.

For an MTT assay in combination with tamoxifen, cells were inoculated into a 6-well plate at 5 × 10⁵ cells/well in 2 mL, and cultured overnight in a 37°C and 5% CO₂ incubator. Transfection was conducted as described above. After 12 h of culturing, cells in the 6-well plate were digested and collected through centrifugation. Cells from each well were counted. Cells were inoculated into a 96-well plate at 1.5 × 10⁴ cells/well in 100 µL, and cultured overnight in a 37°C and 5% CO₂ incubator. Different concentrations of tamoxifen were added, and cells were further cultured for 48 h. An MTT solution (5 mg/mL) was added to each well. Cells were further cultured for 4 h. The resulting culture supernatant in each well was carefully discarded. 150 µL of DMSO was added to each well, and shaking was conducted for 10 min. An absorbance value of each well was measured at a wavelength of 490 nm.

### 3.2 Experimental results

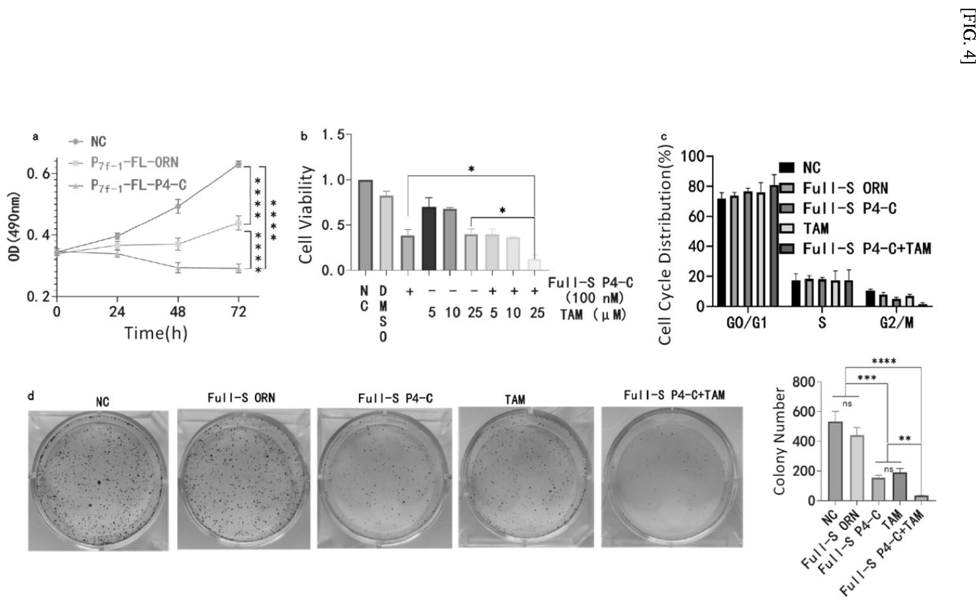

Full-S P4-C (Let-7f-1 Full-S P4-C) at 100 nM significantly inhibited the proliferation of T47D cells. The corresponding inhibitory effect was enhanced over time and was stronger than an inhibitory effect of Full-S ORN without coupled pomalidomide (FIG. 4a).

At a concentration of 100 nM, Full-S P4-C (Let-7f-1 Full-S P4-C) alone resulted in a cell viability of approximately 40% for T47D cells. When tamoxifen was administered alone at 25 µM, the viability of T47D cells was approximately 40%. However, the combination of Full-S P4-C (Let-7f-1 Full-S P4-C) and tamoxifen significantly enhanced the inhibitory effect on T47D cell proliferation, with a cell viability of about 10% (FIG. 4b).

### 4. Experiment on the inhibition of T47D cell colony formation by Full-S P4-C in combination with tamoxifen

### 4.1 Experimental method

Cells were inoculated into a 6-well plate at 5 × 10⁵ cells/well in 2 mL, and cultured overnight in a 37°C and 5% CO₂ incubator. Full-S P4-C (Let-7f-1 Full-S P4-C) and Full-S ORN were transfected using Lipofectamine 2000. For wells treated with tamoxifen alone and wells treated with the combination of tamoxifen and Full-S P4-C (Let-7f-1 Full-S P4-C), after 24 h of culturing, 25 µM tamoxifen was added and cells were further cultured for 24 h. Cells in the 6-well plate were digested and collected through centrifugation. Cells from each well were counted. Then, cells were inoculated into a 6-well plate at 1,000 cells/well and further cultured for three weeks, during which a medium was replaced and a cell status was observed every three days.

### 4.2 Experimental results

Consistent with the MTT assay, Full-S P4-C (Let-7f-1 Full-S P4-C) at 100 nM significantly inhibited the colony formation ability of T47D cells, while Full-S ORN without coupled pomalidomide exhibited relatively low inhibitory efficiency (FIG. 4d).

Compared with tamoxifen alone, the combination of Full-S P4-C (Let-7f-1 Full-S P4-C) and tamoxifen further strengthened the inhibition of colony formation of T47D cells. An inhibitory effect of the combination treatment on colony formation of T47D cells was approximately 5-fold to 6-fold higher than an inhibitory effect of tamoxifen alone (FIG. 4d).

### 5. Experiment on the apoptosis of T47D cells induced by Full-S P4-C in combination with tamoxifen

### 5.1 Experimental method

Apoptosis assay procedure: Cells were inoculated into a 12-well plate at 2 × 10⁵ cells/well to achieve a cell density of 70% to 80%, and then cultured overnight in a 37°C and 5% CO₂ incubator. Transfection was conducted as described in the section 1.1.1. After 24 h of culturing, different concentrations of tamoxifen were added, and cells were further cultured for 48 h. All cells were collected using 0.25% trypsin and washed with PBS. Washed cells were detected using an Annexin V-FITC apoptosis assay kit as follows: The washed cells were resuspended in 195 µL of a binding buffer, 5 µL of Annexin V-FITC was added, and thorough mixing was conducted. Then, 10 µL of propidium iodide (PI) was added, and thorough mixing was conducted gently. The resulting mixture was incubated for 15 min at room temperature in the dark. Apoptosis was detected using a flow cytometer.

### 5.2 Experimental results

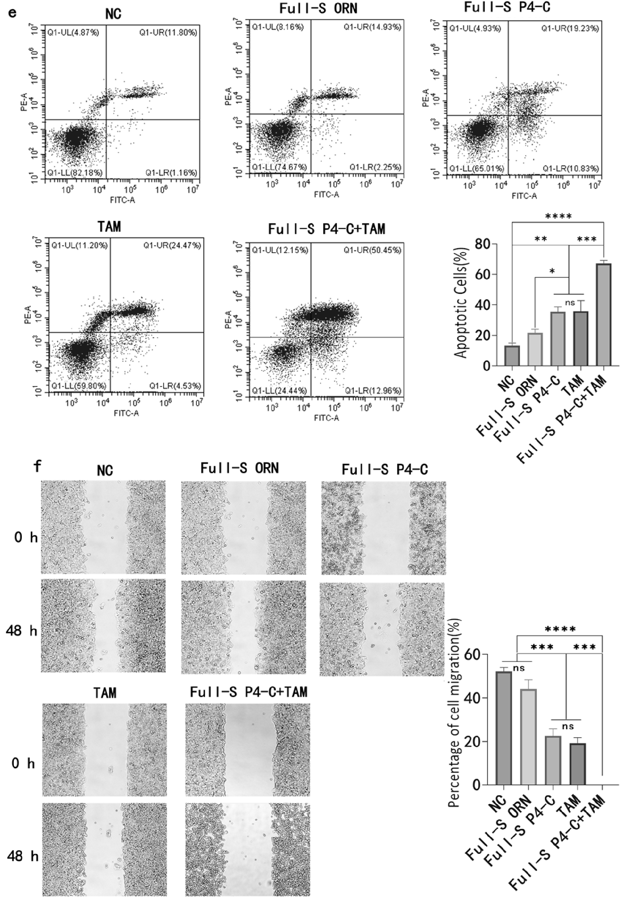

At a concentration of 100 nM, the Full-S P4-C (Let-7f-1 Full-S P4-C) group exhibited superior apoptosis-inducing efficiency in T47D cells to the Full-S ORN group, with a significant difference. Tamoxifen alone at 25 nM achieved comparable apoptosis-inducing efficiency in T47D cells to Full-S P4-C (Let-7f-1 Full-S P4-C), with an apoptosis rate of approximately 30%. The combination of Full-S P4-C (Let-7f-1 Full-S P4-C) and tamoxifen substantially promoted the apoptosis of T47D cells, with an apoptosis rate of about 70% (FIG. 4e).

### 6. Experiment on the cell cycle arrest of T47D cells induced by Full-S P4-C in combination with tamoxifen

### 6.1 Experimental method

Cell cycle assay procedure: Cells were inoculated into a 12-well plate at 2 × 10⁵ cells/well to achieve a cell density of 70% to 80%, and then cultured overnight in a 37°C and 5% CO₂ incubator. Transfection was conducted as described in the section 1.1.1. After 24 h of culturing, different concentrations of tamoxifen were added, and cells were further cultured for 48 h. All cells were collected using 0.25% trypsin, washed with PBS, and fixed with pre-chilled 70% ethanol overnight at 4°C. Fixed cells were then washed once with PBS pre-chilled at 4°C and then detected using a cell cycle assay kit as follows: 500 µL of a staining buffer, 25 µL of a PI staining solution, and 10 µL of RNaseA were added for slowly and fully resuspending cells, and incubation was conducted for 30 min at 37°C in the dark. A cell cycle was detected using a flow cytometer.

### 6.2 Experimental results

When Full-S P4-C (Let-7f-1 Full-S P4-C) was administered alone at 100 nM, T47D cells in the G0/G1 phase increased slightly from 71% to 76%, while cells in the G2/M phase decreased slightly from 10% to 5%. Tamoxifen alone resulted in the same proportions of T47D cells in the G0/G1 phase and in the G2/M phase as Full-S P4-C (Let-7f-1 Full-S P4-C) alone. After the combination treatment, a proportion of T47D cells in the G0/G1 phase increased to 81%, while a proportion of T47D cells in the G2/M phase decreased to less than 1%. Therefore, the combination of Full-S P4-C (Let-7f-1 Full-S P4-C) and tamoxifen effectively arrested T47D cells in the G0/G1 phase (FIG. 4c).

### 7. Experiment on the inhibition of T47D cell migration by Full-S P4-C in combination with tamoxifen

### 7.1 Experimental method

T47D cells were inoculated into a 6-well plate at 5 × 10⁵ cells/well in 2 mL, and cultured overnight in a 37°C and 5% CO₂ incubator. Transfection was conducted as described in the section 1.1.1. Cells were further cultured for 12 h. Culture-Insert was placed in a 6-well plate. Cells were digested, collected through centrifugation, and prepared into a cell suspension with a concentration of 5 × 10⁵ cells/mL. 100 µL of the cell suspension was added to each of two chambers of the Culture-Insert, and incubated at 37°C and 5% CO₂ for 24 h. The Culture-Insert was gently taken out using sterile tweezers. A cell layer was washed with a cell-free medium or PBS to remove cell debris and non-adherent cells. Then, 2 mL of a fresh medium was added (for the tamoxifen experimental group, a tamoxifen-containing medium was added), and cells were further cultured for an appropriate period of time. Cells were imaged using an automated inverted fluorescence microscope at 0 h, 12 h, 24 h, and 48 h, and results were analyzed using ImageJ software.

### 7.2 Experimental results

At a concentration of 100 nM, Full-S P4-C (Let-7f-1 Full-S P4-C) exhibited a stronger ability to inhibit T47D cell migration than Full-S ORN, with a significant difference. Tamoxifen alone at 25 µM achieved a comparable ability to inhibit T47D cell migration to Full-S P4-C (Let-7f-1 Full-S P4-C), with a T47D cell migration rate of approximately 20%. The combination of Full-S P4-C (Let-7f-1 Full-S P4-C) and tamoxifen significantly enhanced the inhibition of T47D cell migration, reducing the migration rate to 0 (FIG. 4f).

### Experimental Example 2 Targeted degradation of a Lin28B protein in H1299 cells and subsequent bioactivity evaluation experiments

### 1. Experiment for evaluating a degradation effect on the Lin28B protein

### 1.1 Experimental method

Cell culture and transfection: The cell culture and transfection were conducted as described in the relevant sections of Experimental Example 1, except that the transfection reagent was replaced with Lipofectamine 3000.

Detection of Lin28B protein expression levels and ubiquitination effects: Experimental procedures for detecting the Lin28B protein expression levels and ubiquitination effects were the same as those described in the relevant sections of Experimental Example 1.

### 1.2 Experimental results

### Evaluation of degradation effects of Lin28B in H1299 cells:

After being coupled to pomalidomide through a PEG4 linker, all sequences capable of binding to the Lin28B protein, Let-7b P4-C (Let-7b Full-S P4-C), Let-7d P4-C (Let-7d Full-S P4-C), Let-7f-1 P4-C (Let-7f-1 Full-S P4-C), Let-7g P4-C (Let-7g Full-S P4-C), and Let-7i P4-C (Let-7i Full-S P4-C), all exhibited considerable degradation activity. The Let-7f-1 sequence (SEQ ID No. 13) was selected for subsequent screening of linker lengths and E3 Ub ligase ligands (FIG. 1b).

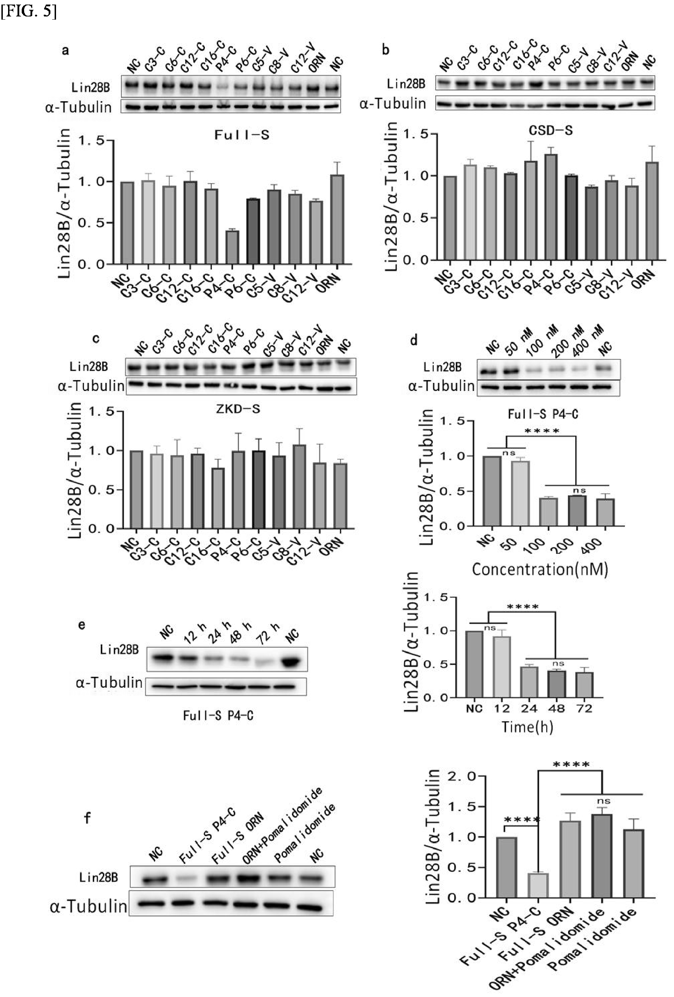

Based on Full-S sequences capable of binding to both CSD and ZKD, Full-S P4-C exhibited pronounced degradation effects under conditions of 48 h and 100 nM. All samples produced from CSD-S sequences capable of binding only to CSD and ZKD-S sequences capable of binding only to ZKD did not show significant degradation activity against the Lin28B protein (FIG. 5a, FIG. 5b, and FIG. 5c). Under the condition of 48 h, Full-S P4-C achieved a maximum degradation effect at 100 nM (FIG. 5d). At a transfection concentration of 100 nM, Full-S P4-C achieved a maximum degradation effect at 24 h (FIG. 5e). A Full-S P4-C experimental group exhibited a better degradation effect on Lin28B than a Full-S ORN experimental group without coupled pomalidomide and a Full-ORN/pomalidomide mixture group. Moreover, an experimental group treated with pomalidomide alone did not lead to significant degradation of the Lin28B protein. It indicated that the degradation of Lin28B mediated by Full-S P4-C was specific degradation mediated jointly by Full-S ORN and pomalidomide that were coupled, rather than non-specific degradation mediated by any single one of the aforementioned components alone (FIG. 5f). According to a ubiquitination experiment, a Full-S P4-C (Let-7f-1 Full-S P4-C) treatment effectively increased the polyubiquitination of the Lin28B protein in H1299 cells. Further, the addition of the protease inhibitor MG132 significantly attenuated the degradation on Lin28B, indicating that the Full-S P4-C-mediated degradation of the Lin28B protein was achieved through a proteasome pathway.

### 2. Experiment on miRNA let-7 level changes after Full-S P4-C-mediated degradation of Lin28B

### 2.1 Experimental method

Cell inoculation: H1299 cells were inoculated into a 24-well plate at 6 × 10⁴ cells/well to achieve a cell density of 70% to 80%, and then cultured overnight in a 37°C and 5% CO₂ incubator. Transfection was conducted as described in the section 1.1.1 of Experimental Example 1.

Detection of miRNA let-7 expression: Experimental procedures for detecting a let-7 expression level were the same as those described in the relevant sections of Experimental Example 1.

### 2.2 Experimental results

miRNA let-7 expression levels were shown in FIG. 3. As the Lin28B protein was degraded, the expression levels of all mature let-7 members were upregulated by 1.7-fold approximately, and the expression level of mirR-21 remained unchanged. It indicated that the targeted degradation of Lin28B by Full-S P4-C (Let-7f-1 Full-S P4-C) specifically led to an increase in let-7 expression levels (FIG. 3e). Under the condition of 48 h, as the concentration of Full-S P4-C increased, the expression level of Let-7g was gradually upregulated, and reached a maximum value at 100 nM (FIG. 3f). At a transfection concentration of 100 nM, as the incubation time extended, the expression level of Let-7g increased slightly at 24 h and significantly increased to a maximum value at 48 h (FIG. 3g). Under the same transfection concentration and time, the Full-S P4-C experimental group exhibited a better effect on increasing let-7g levels than the Full-S ORN experimental group without coupled pomalidomide and the Full-ORN/pomalidomide mixture group, while the experimental group treated with pomalidomide alone did not show a significant increase in let-7g levels (FIG. 3h).

### 3. Experiment on the inhibition of proliferation and colony formation of H1299 cells

### 3.1 Experimental method: MTT and colony formation assays were conducted as described in the relevant sections of Experimental Example 1.

### 3.2 Experimental results

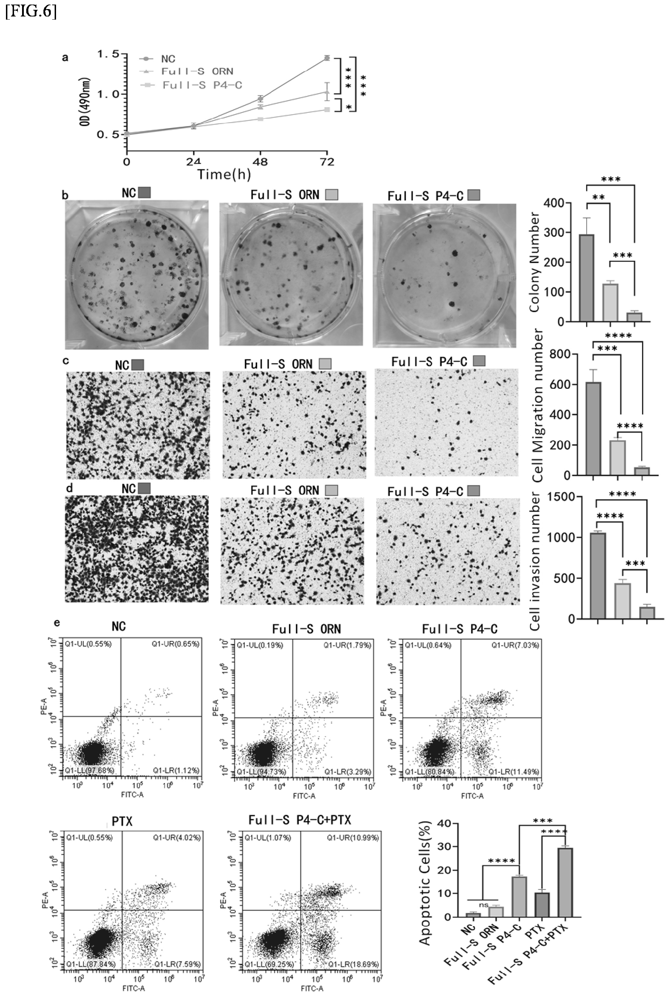

Full-S P4-C (Let-7f-1 Full-S P4-C) at 100 nM inhibited the proliferation of H1299 cells, with a stronger inhibitory effect than Full-S ORN without coupled pomalidomide (FIG. 6a).

Consistent with the MTT assay, Full-S P4-C (Let-7f-1 Full-S P4-C) at 100 nM significantly inhibited the colony formation ability of H1299 cells, while Full-S ORN without coupled pomalidomide exhibited relatively low inhibitory efficiency (FIG. 6b).

### 4. Experiment on the inhibition of migration and invasion of H1299 cells

### 4.1 Experimental method

Experimental procedures: Cells were inoculated into a 6-well plate at 4 × 10⁵ cells/well in 2 mL, and cultured overnight in a 37°C and 5% CO₂ incubator. Transfection was conducted as described in the section 1.2. Cells were further cultured for 48 h. A serum-free medium and Matrigel were thoroughly mixed at a ratio of 1:5, then added to an upper chamber of 24-Transwell (at 100 µL/well), and incubated in a 37°C incubator for 1 h to 2 h. After transfection and 48 h of culturing, resulting cells were digested, counted, and prepared into a cell suspension (100,000 cells/mL). 200 µL of the cell suspension was added to each well. 500 µL of a medium including 10% fetal bovine serum (FBS) was added to a lower chamber. Incubation was conducted in a 37°C incubator for 24 d. Cells were washed twice with PBS, fixed with paraformaldehyde, stained with a 0.1% crystal violet staining solution, and washed twice with PBS. Cells on the upper surface were removed using a cotton swab. Stained cells were observed and photographed under a microscope. A cell migration assay was the same as the cell invasion assay, except that Matrigel was not added.

### 4.2 Experimental results

Full-S P4-C (Let-7f-1 Full-S P4-C) at 100 nM significantly inhibited the migration and invasion of H1299 cells, with a superior inhibitory effect to Full-S ORN (FIG. 6c and FIG. 6d).

### 5. Experiment on the apoptosis of H1299 cells induced by Full-S P4-C in combination with paclitaxel

### 5.1 Experimental method: An apoptosis assay was conducted as described in the relevant section of Experimental Example 1.

### 5.2 Experimental results

At a concentration of 100 nM, the Full-S P4-C (Let-7f-1 Full-S P4-C) group exhibited superior apoptosis-inducing efficiency in H1299 cells to the Full-S ORN group, with a significant difference. Paclitaxel alone at 50 µM achieved an apoptosis-inducing efficiency of about 10% in H1299 cells. The combination of Full-S P4-C (Let-7f-1 Full-S P4-C) and paclitaxel substantially promoted the apoptosis of H1299 cells, with an apoptosis rate of about 30% (FIG. 6e).

### Experimental Example 3 Targeted degradation of a Lin28B protein in Huh7 cells and subsequent bioactivity evaluation experiments

### 1. Experiment for evaluating a degradation effect on the Lin28B protein

### 1.1 Experimental method

Cell culture and transfection: The cell culture and transfection were conducted as described in the relevant sections of Experimental Example 1, except that the transfection reagent was replaced with Lipofectamine 3000.

Detection of Lin28B protein expression levels and ubiquitination effects: Experimental procedures for detecting the Lin28B protein expression levels and ubiquitination effects were the same as those described in the relevant sections of Experimental Example 1.

### 1.2 Experimental results

### Evaluation of degradation effects of Lin28B in Huh7 cells:

After being coupled to pomalidomide through a PEG4 linker, all sequences capable of binding to the Lin28B protein exhibited varying degradation activities. Let-7b P4-C (Let-7b Full-S P4-C) and Let-7f-1 P4-C (Let-7f-1 Full-S P4-C) showed the optimal degradation activity, and could reduce the Lin28B expression level to 10% or less. The Let-7b sequence (SEQ ID No. 7) was selected for subsequent screening of linker lengths and E3 Ub ligase ligands (FIG. 1c).

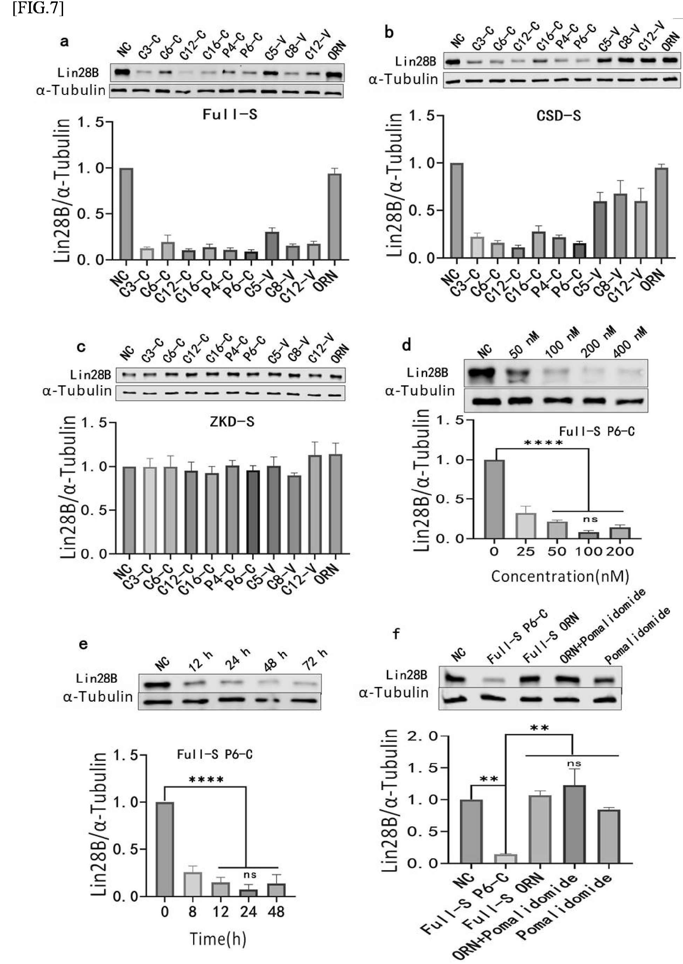

Under conditions of 48 h and 100 nM, PROTAC molecules produced from sequences capable of binding to both CSD and ZKD of the Lin28B protein all exhibited significant degradation activity against the Lin28B protein. Let-7b Full-S C12-C, Let-7b Full-S P4-C, and Let-7b Full-S P6-C showed the optimal activities, and could reduce the Lin28B expression level to approximately 10% (FIG. 7a). Based on sequences capable of binding only to CSD of the Lin28B protein, Let-7b CSD-S C3-C, Let-7b CSD-S C6-C, Let-7b CSD-S C12-C, Let-7b CSD-S C16-C, Let-7b CSD-S P4-C, and Let-7b CSD-S P6-C all could effectively degrade Lin28B, and could reduce the expression level of Lin28B to approximately 10% to 20% (FIG. 7b). All samples produced from sequences capable of binding only to ZKD of the Lin28B protein did not show significant degradation activity against the Lin28B protein (FIG. 7c).

Subsequently, Let-7b Full-S P6-C was selected for evaluation of time-dependent and concentration-dependent effects. Under the condition of 48 h, the Let-7b Full-S P6-C molecule resulted in significant degradation for Lin28B at a concentration of 25 nM, and achieved a maximum degradation effect at 100 nM (FIG. 7d). At a transfection concentration of 100 nM, the Let-7b Full-S P6-C molecule led to significant degradation of Lin28B at 8 h, and still maintained high degradation activity even at 48 h (FIG. 7e). A Let-7b Full-S P6-C experimental group exhibited a better degradation effect on Lin28B than a Full-S ORN experimental group without coupled pomalidomide and a Full-S ORN/pomalidomide mixture group. Moreover, an experimental group treated with pomalidomide alone did not lead to significant degradation of the Lin28B protein. It indicated that the degradation of Lin28B mediated by Let-7b Full-S P6-C was specific degradation mediated jointly by Full-S ORN and pomalidomide that were coupled, rather than non-specific degradation mediated by any single one of the aforementioned components alone (FIG. 7f). According to a ubiquitination experiment, a Let-7b Full-S P6-C treatment increased the polyubiquitination of the Lin28B protein in Huh-7 cells. The addition of the proteasome inhibitor MG132 substantially attenuated the degradation effect of Let-7b Full-S P6-C, indicating that Let-7b Full-S P6-C mediated the degradation of Lin28B through a proteasome pathway.

### Experimental Example 4 In vivo evaluation of anti-tumor activities of PROTAC molecules

### 1. Experimental method

In this experiment, 6-week-old female BALB/c nude mice weighing 16 g to 20 g were selected and housed in a barrier environment. The mice were ordered one week in advance for acclimatization to the experimental environment. The mice were provided with sufficient food and water and a normal circadian rhythm. T47D cells cultured under normal proliferation conditions were digested with 0.25% trypsin at 37°C for 3 min. A serum-containing medium was then added to terminate the digestion. The cells were washed with 1× PBS and then resuspended in a serum-free 1640 medium. Matrigel was added at an equal amount to the medium, and thorough mixing was conducted by pipetting up and down. Each mouse was subcutaneously injected with 1 × 10⁷ T47D cells. To stimulate tumor growth, 0.1 mL of β-estradiol 17-valerate (5 mg/mL in sesame oil) was subcutaneously injected into a scapular region of each mouse. A tumor growth status was monitored regularly. When a tumor volume reached approximately 50 mm³, T47D tumor-bearing mice were randomly divided into the following 5 groups: A first group: Mice were intratumorally injected with 0.9% normal saline. A second group: Mice were intratumorally injected with Full-S ORN (6 nmol/mouse). A third group: Mice were intratumorally injected with Full-S P4-C (Let-7f-1 Full-S P4-C) (6 nmol/mouse). A fourth group: Mice were intragastrically administered tamoxifen (5 mg/kg, dissolved in corn oil). A fifth group: Mice were intratumorally injected with Full-S P4-C (Let-7f-1 Full-S P4-C) (6 nmol/mouse) and intragastrically administered tamoxifen (5 mg/kg). The nucleic acid was administered a total of 8 times respectively on day 1, day 4, day 7, day 10, day 13, day 16, day 19, and day 22. The tamoxifen was administered a total of 8 times respectively on day 2, day 5, day 8, day 11, day 14, day 17, day 20, and day 23. During the experimental period, a body weight and a tumor size of each nude mouse were monitored once every three days. The tumor size was monitored by measuring a long diameter a and a short diameter b of a tumor. A tumor volume was calculated according to a formula 1/2 × a × b². 2 d after the last administration, tumors were collected, photographed, and weighed. Lin28A protein levels in tumors were characterized by WB. Paraffin sections of tumors were prepared and subjected to immunohistochemical (IHC) staining to characterize the distribution of the Lin28A protein at the tumor tissue level. In this experiment, hematoxylin & eosin (H&E) staining was also conducted on tissue sections to analyze pathological conditions in tumor tissues.

### 2. Experimental results

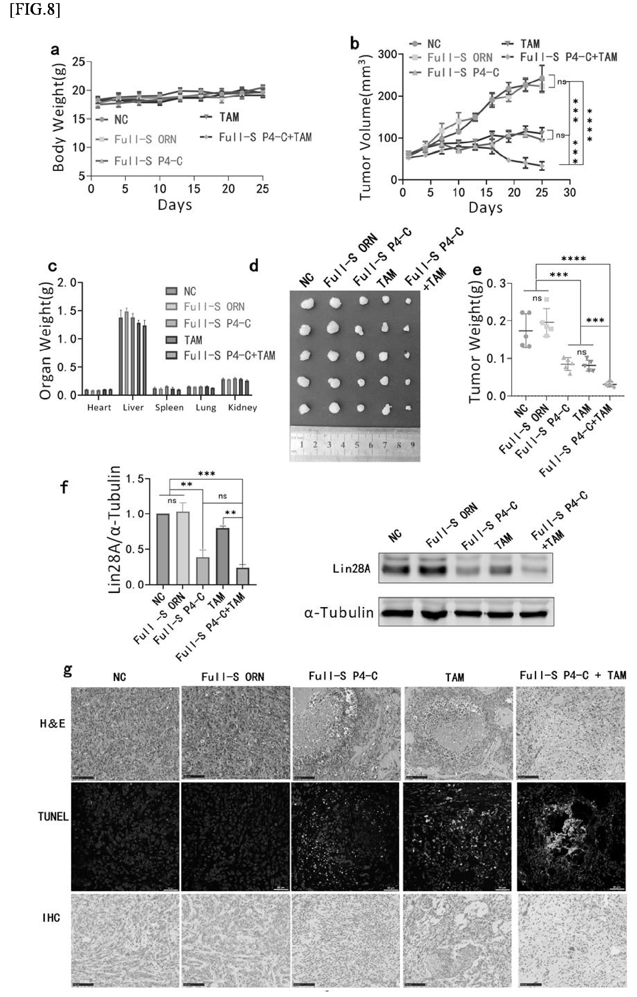

During the administration period, the administration groups showed no significant difference from the NC group in terms of body weights, and nude mice in each administration group had normal growth and living statuses. It indicated that the PROTAC molecule Full-S P4-C (Let-7f-1 Full-S P4-C) and tamoxifen exhibited no obvious toxicity (FIG. 8a). There was no significant difference between the Full-S ORN group and the NC group in terms of the growth of breast cancer. Both Full-S P4-C (Let-7f-1 Full-S P4-C) alone and tamoxifen alone effectively inhibited the growth of breast cancer. Compared with the single-agent treatment, the combination treatment further inhibited the growth of breast cancer, and led to a tendency of tumor regression (FIG. 8b). According to tumor dissection and weighing results for breast cancer, the Full-S ORN group had a similar tumor weight to the NC group, the Full-S P4-C (Let-7f-1 Full-S P4-C) single-agent treatment group and the tamoxifen single-agent treatment group had a significantly smaller tumor weight than the NC group, and the combination treatment group had a smaller tumor weight than the single-agent treatment groups (FIG. 8d and FIG. 8e). The administration groups showed no marked difference from the NC group in terms of heart, liver, spleen, lung, and kidney weights (FIG. 8c), further demonstrating that Full-S P4-C (Let-7f-1 Full-S P4-C) and tamoxifen had no obvious toxicity. According to WB results, Full-S P4-C (Let-7f-1 Full-S P4-C) effectively degraded the Lin28A protein in breast cancer tissues (FIG. 8f). H&E staining results revealed that the NC group and the Full-S ORN group exhibited typical morphological structures of tumor tissues. The Full-S P4-C (Let-7f-1 Full-S P4-C) and tamoxifen single-agent treatment groups had obvious phenotypes of cell death and nuclear pyknosis and a much smaller cancer cell density than the NC group and the Full-S ORN group. Moreover, the Full-S P4-C (Let-7f-1 Full-S P4-C)-tamoxifen combination treatment group had a much smaller cancer cell density than the single-agent treatment groups.

According to TUNEL staining results, no obvious apoptotic fluorescence signals were observed in the NC group and the Full-S ORN group, obvious apoptotic fluorescence signals were observed in both the Full-S P4-C (Let-7f-1 Full-S P4-C) single-agent treatment group and the tamoxifen single-agent treatment group, and extensive apoptotic fluorescence signals were observed in the combination treatment group. According to IHC results, both the NC group and the Full-S ORN group exhibited a strongly positive phenotype for Lin28A. The Full-S P4-C (Let-7f-1 Full-S P4-C) single-agent treatment group exhibited significantly weakened positivity for Lin28A. Although the tamoxifen single-agent treatment group underwent morphological changes in tissues, a strongly positive phenotype for Lin28A was still maintained. A positive phenotype was further weakened in the combination treatment group, which was consistent with the WB results for proteins in tumor tissues (FIG. 8g). In summary, the above results reveal that the PROTAC molecule Full-S P4-C (Let-7f-1 Full-S P4-C) can effectively inhibit the growth of breast cancer, and can exert an enhanced synergistic anti-tumor effect when used in combination with tamoxifen.

## Claims

1. An oligonucleotide-based proteolysis-targeting chimera (PROTAC) molecule for targeted degradation of a Lin28A/B protein, wherein the oligonucleotide-based PROTAC molecule is produced by coupling an oligonucleotide sequence capable of binding to the Lin28A/B protein to a ligand capable of promoting ubiquitination of the Lin28A/B protein through a linker.

2. The oligonucleotide-based PROTAC molecule for targeted degradation of the Lin28A/B protein according to claim 1, wherein the ligand capable of promoting ubiquitination of the Lin28A/B protein is pomalidomide, a von Hippel-Lindau (VHL) ligand VH032, or a derivative or an analog thereof;
a chemical structural formula of the pomalidomide is shown in a formula I: 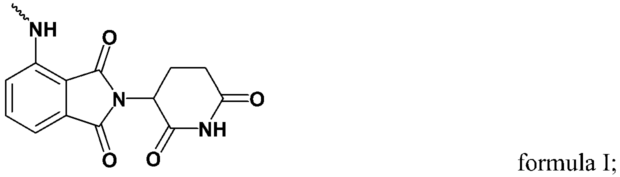
a structural formula of the VHL ligand VH032 is shown in a formula II: 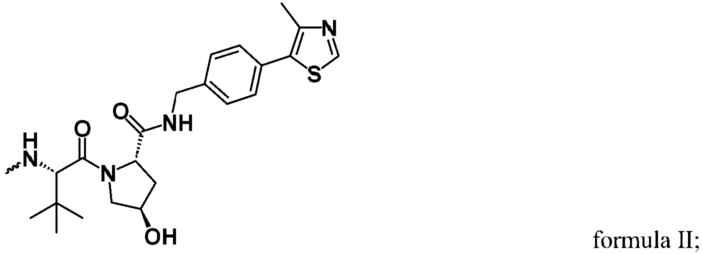 and
a structural formula of the derivative or the analog thereof is shown in a formula III:

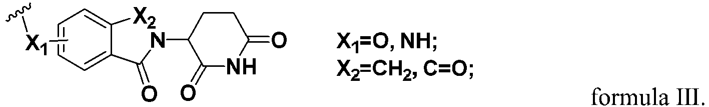

3. The oligonucleotide-based PROTAC molecule for targeted degradation of the Lin28A/B protein according to claim 1, wherein the oligonucleotide sequence capable of binding to the Lin28A/B protein is an oligonucleotide sequence that binds to both a cold shock domain (CSD) and a zinc knuckle domain (ZKD) of the Lin28A/B protein, or an oligonucleotide sequence that binds only to the CSD of the Lin28A/B protein, or an oligonucleotide sequence that binds only to the ZKD of the Lin28A/B protein; and preferably, a backbone of the oligonucleotide sequence is thio-modified or modified or substituted with different nucleotide analogs, comprising, but not limited to following modified nucleobases: 5-methylcytosine, 6-methyladenine, pseudouracil, or inosine; or a nucleotide having a methoxy, methylethoxy, or fluoro modification at a 2' position of a sugar ring, or a locked nucleic acid.

4. The oligonucleotide-based PROTAC molecule for targeted degradation of the Lin28A/B protein according to claim 3, wherein the oligonucleotide sequence that binds to both the CSD and the ZKD of the Lin28A/B protein is any nucleotide sequence selected from SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, or SEQ ID No. 23; the oligonucleotide sequence that binds only to the CSD of the Lin28A/B protein is any nucleotide sequence selected from SEQ ID No. 2, SEQ ID No. 4, SEQ ID No. 6, SEQ ID No. 8, SEQ ID No. 10, SEQ ID No. 12, SEQ ID No. 14, SEQ ID No. 16, SEQ ID No. 18, SEQ ID No. 20, SEQ ID No. 22, or SEQ ID No. 24; or the oligonucleotide sequence that binds to both the CSD and the ZKD of the Lin28A/B protein or the oligonucleotide sequence that binds only to the CSD of the Lin28A/B protein is a nucleotide sequence that has a homology of 80% or more with the above respective nucleotide sequences and still retains a function or an activity of binding to the Lin28A/B protein;
the oligonucleotide sequence that binds only to the ZKD of the Lin28A/B protein is any nucleotide sequence selected from (1) to (12):
(1): 5'-GGGAGAU-3'; (2): 5'-GGGAGAU-3'; (3): 5'-AUGGGAU-3'; (4): 5'-GGAAGAU-3'; (5): 5'-GGGAGUU-3'; (6): 5'-AGGAGGU-3'; (7): 5'-AGGAGAU-3'; (8): 5'-AGGAGAU-3'; (9): 5'-UGGAGAU-3'; (10): 5'-AGGAGAU-3'; (11): 5'-UGGAGAU-3'; and (12): 5'-AGAAGAU-3';
preferably, any of the above oligonucleotide sequences is subjected to a backbone thio modification.

5. The oligonucleotide-based PROTAC molecule for targeted degradation of the Lin28A/B protein according to any one of claims 1 to 4, wherein a ligand molecule targeting a tumor cell is linked to a 5' terminus of the oligonucleotide sequence; and the ligand molecule comprises, but is not limited to, folic acid, a polypeptide, or a nucleic acid aptamer.

6. The oligonucleotide-based PROTAC molecule for targeted degradation of the Lin28A/B protein according to claim 1, wherein the linker is selected from one or more of alkylene, alkoxy, alkylamino, alkylthio, amido, a carbamate linkage, or a triazole derivative;
preferably, the linker is selected from any one of compounds shown in the following general formulas: 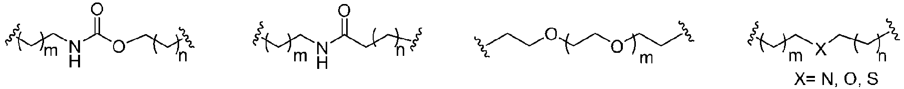 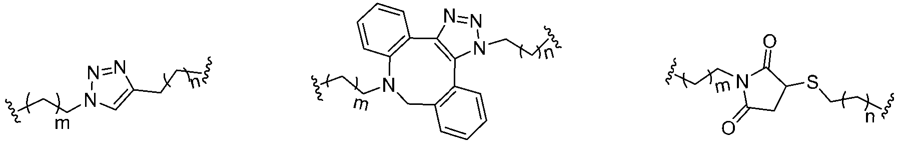
wherein m or n is any integer from 0 to 10; and
more preferably, the linker comprises, but is not limited to, any one of C3, C5, C6, C8, C12-1, C12-2, C16, P4, or P6, wherein a structural formula of C3 is as shown in a formula IV, a structural formula of C5 is as shown in a formula V, a structural formula of C6 is as shown in a formula VI, a structural formula of C8 is as shown in a formula VII, a structural formula of C12-1 is as shown in a formula VIII, a structural formula of C12-2 is as shown in a formula IX, a structural formula of C16 is as shown in a formula X, a structural formula of P4 is as shown in a formula XI, and a structural formula of P6 is as shown in a formula XII:

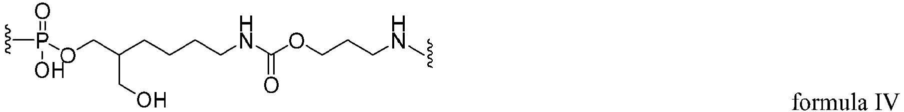

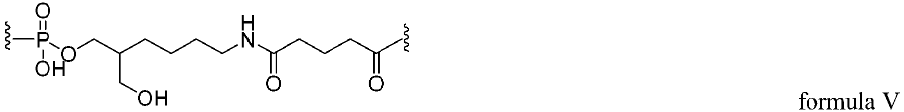

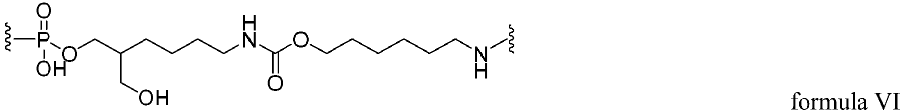

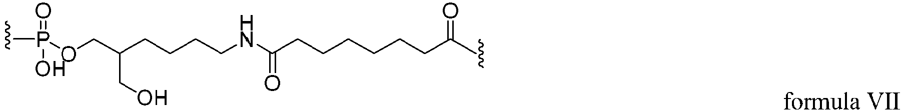

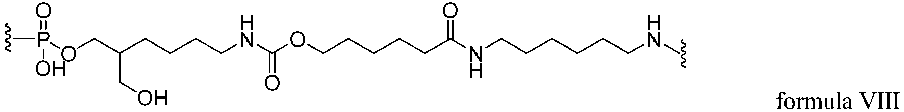

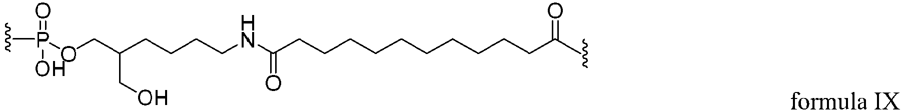

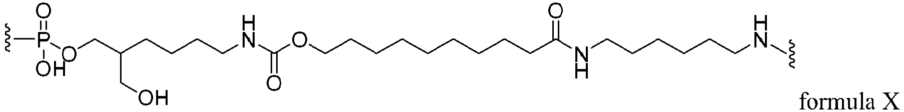

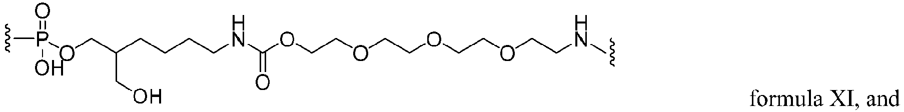

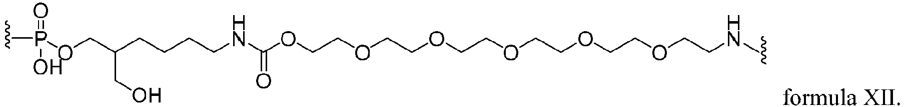

7. A method for preparing the oligonucleotide-based PROTAC molecule for targeted degradation of the Lin28A/B protein according to any one of claims 1 to 4, comprising:
(1) linking a CRBN or VHL ligand to any linker of different types or different lengths, and conducting an activation reaction to produce an N-hydroxysuccinimide (NHS) active ester of the CRBN or VHL ligand with a linker; and
(2) coupling the oligonucleotide sequence that is capable of binding to the Lin28A/B protein and comprises an amino group at a 3' terminus to the NHS active ester of the CRBN or VHL ligand with the linker obtained in the step (1).

8. The method according to claim 7, wherein the step (2) comprises: dissolving the oligonucleotide sequence that is capable of binding to the Lin28A/B protein and comprises the amino group at the 3' terminus in phosphate buffered saline (PBS) to produce a mixed solution 1; dissolving the NHS active ester of the CRBN or VHL ligand with the linker in dimethyl sulfoxide (DMSO) to produce a mixed solution 2; and mixing the mixed solution 1 and the mixed solution 2 to allow a coupling reaction, and purifying a reaction product.

9. A use of the oligonucleotide-based PROTAC molecule for targeted degradation of the Lin28A/B protein according to any one of claims 1 to 4, or a combination of the oligonucleotide-based PROTAC molecule with a small-molecule anti-tumor drug, in preparation of a drug or formulation for targeted degradation of the Lin28A/B protein, wherein preferably, the small-molecule anti-tumor drug is tamoxifen or paclitaxel.

10. A use of the oligonucleotide-based PROTAC molecule for targeted degradation of the Lin28A/B protein according to any one of claims 1 to 4, or a combination of the oligonucleotide-based PROTAC molecule with a small-molecule anti-tumor drug or macromolecular antibody anti-tumor drug, in preparation of a drug or formulation for an anti-tumor therapy, wherein preferably, the Lin28A/B protein is overexpressed in a tumor cell targeted by the anti-tumor therapy; and the small-molecule anti-tumor drug is tamoxifen or paclitaxel.
